# EUROPEAN PATENT SPECIFICATION

(11) **EP 4 466 379 B1**
(45) Date of publication and mention of the grant of the patent: **09.12.2026**
(21) Application number: 23743879.1
(22) Date of filing: 18.01.2023
(51) Int. Cl.: C12Q 1/6876, C12Q 1/6844, C12Q 1/6883, C12Q 1/6888

(54) **SELECTION METHOD FOR DOMESTIC ANIMAL BREEDING**
AUSWAHLVERFAHREN FÜR DIE HAUSTIERZUCHT
PROCÉDÉ DE SÉLECTION POUR L'ÉLEVAGE DOMESTIQUE D'ANIMAUX

(30) Priority: 18.01.2022 US 202263300489 P
(43) Date of publication of application: 27.11.2024
(73) Proprietor: THE PENN STATE RESEARCH FOUNDATION, University Park, PA 16802 (US)
(72) Inventor: DECHOW, Chad, University Park, Pennsylvania 16802 (US)
(74) Representative: J A Kemp LLP
(86) International application number: PCT/US2023/060834
(87) International publication number: WO 2023/141462

(56) References cited:
- WO-A1-2009/127211
- US-A1- 2015 344 974
- US-A1- 2020 375 156
- KUTZER T ET AL: "CURRENT STATE OF DEVELOPMENT OF GENOME ANALYSIS IN LIVESTOCK", CURRENT GENOMICS, BENTHAM SCIENCE PUBLISHERS LTD, NL, vol. 4, no. 6, 1 January 2003 (2003-01-01), pages 487 - 525, XP009052911, ISSN: 1389-2029, DOI: 10.2174/1389202033490286
- TANG ZHEN ZHI ET AL: "Muscle weakness in myotonic dystrophy associated with misregulated splicing and altered gating of CaV1.1 calcium channel", HUMAN MOLECULAR GENETICS, vol. 21, no. 6, 2 December 2011 (2011-12-02), pages 1312 - 1324, XP093021915, ISSN: 0964-6906, DOI: 10.1093/hmg/ddr568
- DECHOW C.D. ET AL: "Identification of a putative haplotype associated with recumbency in Holstein calves", JDS COMMUNICATIONS, vol. 3, no. 6, 1 November 2022 (2022-11-01), pages 412 - 415, XP093345469, ISSN: 2666-9102, DOI: 10.3168/jdsc.2022-0224
- AL-KHUDHAIR A ET AL: "New mutation within a common haplotype is associated with calf muscle weakness in Holsteins", JOURNAL OF DAIRY SCIENCE, ELSEVIER, AMSTERDAM, NL, vol. 107, no. 6, 20 January 2024 (2024-01-20), pages 3768 - 3779, XP087534622, ISSN: 0022-0302, [retrieved on 20240120], DOI: 10.3168/JDS.2023-24121
- DUCHESNE A., EGGEN A.: "Radiation hybrid mapping of genes and newly identified microsatellites in candidate regions for bovine arthrogryposis–palatoschisis and progressive ataxia based on comparative data from man, mouse and rat", JOURNAL OF ANIMAL BREEDING AND GENETICS, BLACKWELL WISSENSCHAFTS VERLAG, BERLIN, DE, vol. 122, no. s1, 1 April 2005 (2005-04-01), DE , pages 28 - 35, XP093093881, ISSN: 0931-2668, DOI: 10.1111/j.1439-0388.2005.00493.x
- GóMEZ ENRIQUE; MUñOZ MARTA; GATIEN JULIE; CARROCERA SUSANA; MARTíN-GONZáLEZ DAVID; SALVETTI PASCAL: "Metabolomic identification of pregnancy-specific biomarkers in blood plasma of BOS TAURUS beef cattle after transfer of in vitro produced embryos", JOURNAL OF PROTEOMICS, ELSEVIER, AMSTERDAM, NL, vol. 225, 20 June 2020 (2020-06-20), AMSTERDAM, NL , XP086219904, ISSN: 1874-3919, DOI: 10.1016/j.jprot.2020.103883

## Description

### CROSS-REFERENCE TO RELATED APPLICATIONS

This application claims the benefit of U.S. Provisional Application 63/300,489 filed January 18, 2022.

### GOVERNMENT SUPPORT

This invention was made with government support under Hatch Act Project No. PEN04672 awarded by the United States Department of Agriculture. The Government has certain rights in the invention.

### SEQUENCE LISTING

This application includes a sequence listing in XML format titled "2023-01-17_900905.00047_WIPO Sequence listing.xml", which is 33,063 bytes in size and was created on January 17, 2023. The sequence listing is electronically submitted with this application via Patent Center.

### FIELD

The present disclosure relates to the fields of animal husbandry, animal breeding, and livestock management by using genetic analysis to identify haplotypes and mutations linked to undesirable traits.

### BACKGROUND

Selective breeding (also called artificial selection) is the process by which humans use animal and plant breeding to develop particular phenotypic traits (characteristics) by choosing which specific animals or plants, males and females, will sexually reproduce to produce offspring. In animal breeding, techniques such inbreeding, linebreeding, and outcrossing are utilized. The deliberate exploitation of selective breeding to produce desired traits has become very common in agriculture and experimental biology.

Dairy cows are significant investments for dairy farmers, and enormous efforts, such as systematic animal breeding programs and artificial insemination, have been and continue to be invested in ensuring that the animals have high and sustained productivity, that the milk produced is of high quality or has desired composition, and that the cows are healthy and fertile.

Traditional breeding techniques involve the studying of sire progenies, and evaluating their milk production, health and fertility ratings (transmitting abilities) to guide further breeding. This standard technique requires years to evaluate the true genetic value by progeny testing each bull. Many cows must be bred and give birth to offspring. The females must be raised, bred, allowed to give birth and finally milked for a length of time to measure their phenotypic traits. Bulls with high performing daughters are then returned to active service and are at least five years old. Those with poor performing daughters are culled with substantial financial loss because there is no opportunity to sell semen and recover the investment of raising the bull.

Furthermore, selection based purely on phenotypic characteristics does not efficiently take into account genetic variability caused by complex gene action and interactions, and the effect of environmental and developmental variants. There is thus a need for a method of genetically evaluating cattle to enable breeders to more accurately select animals at both the phenotypic and the genetic level early in life.

Marker-assisted selection can lower the high cost and reduce the extended time commitment of progeny testing used to improve a herd, since young animals could be evaluated immediately after birth or even prior to birth for the presence/absence of the key markers, and young animals, such as bulls, that are determined by genetic testing to have undesirable markers would never be progeny tested and may never be bred. Therefore, there is a need in the art for faster, more accurate genetic testing of domestic animals such as dairy cattle.

Also mentioned is WO 2009/127211 A1 which refers to methods and kits for determining spinal dysmyelination.

### SUMMARY

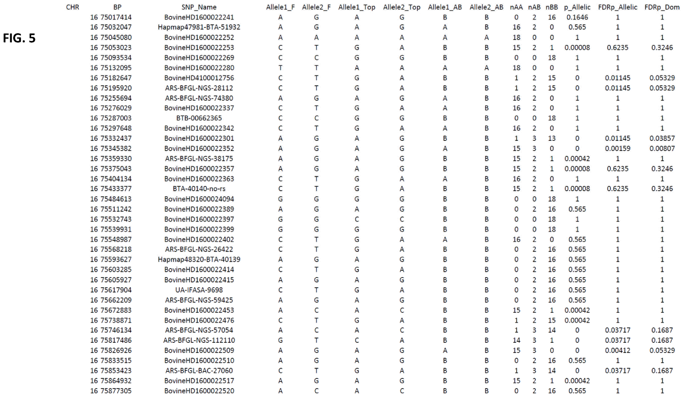

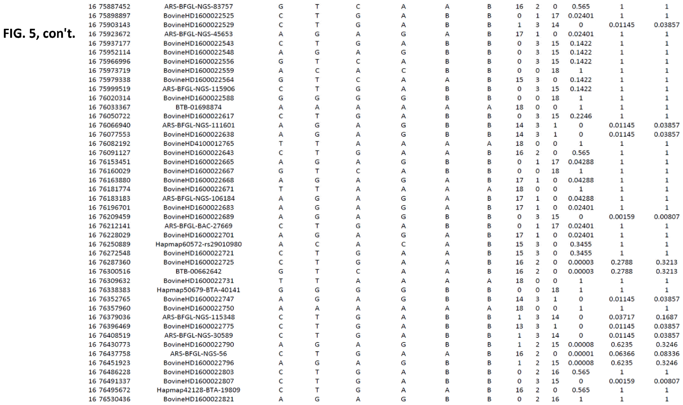

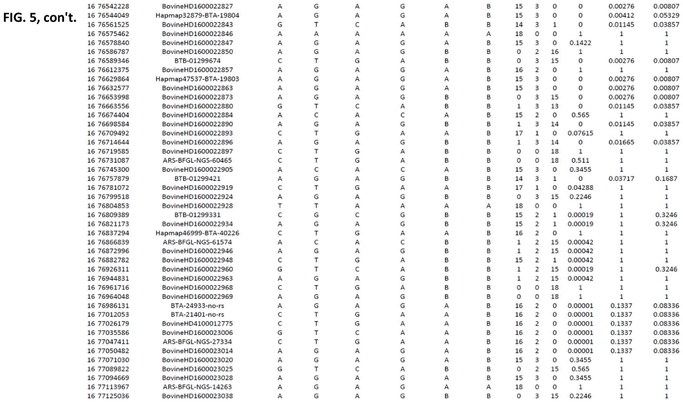

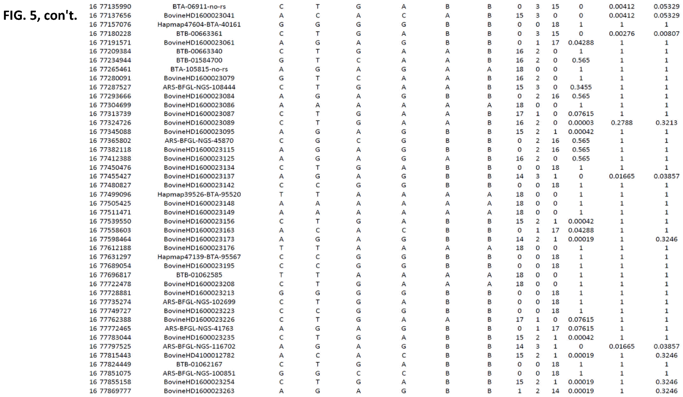

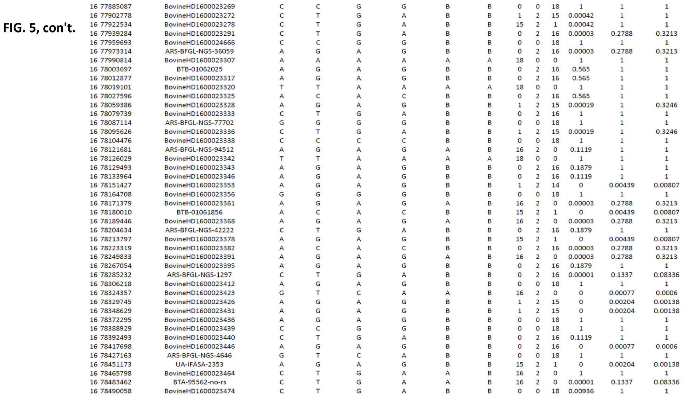

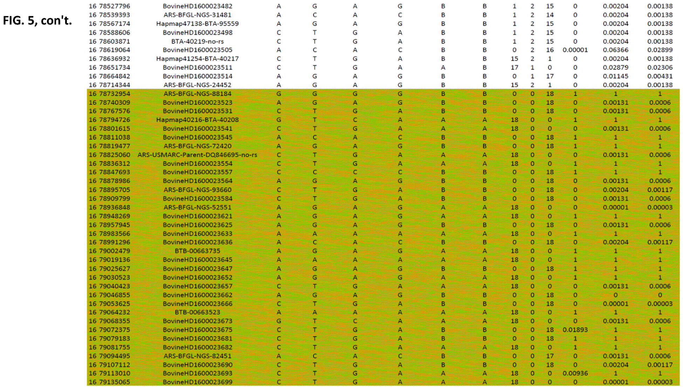

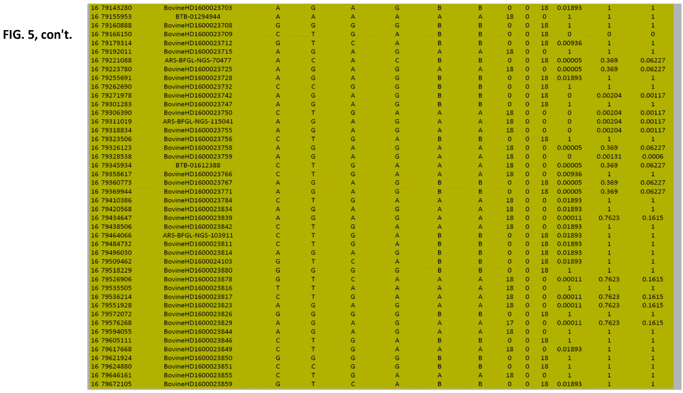

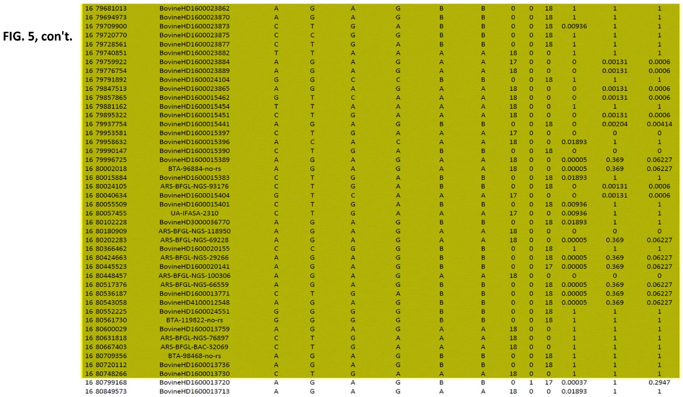

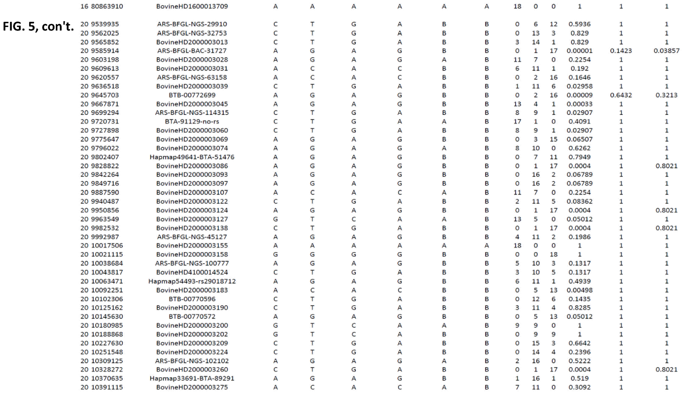

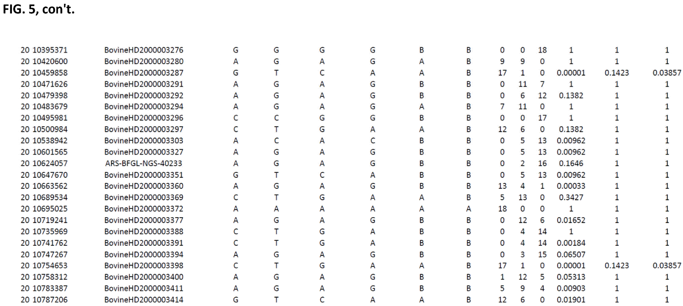

Disclosed herein are methods, systems and compositions for identifying domestic livestock, such as cattle (e.g., *Bos taurus, Bos indicus),* having genetic traits associated with a motor impairment phenotype. Hence, the present invention provides a method comprising:
(a) generating genetic data from chromosomal DNA at position 78732954 to 80748266 bp on chromosome 16, or a fragment thereof, wherein the chromosomal DNA is present in a sample that has been obtained from a domestic bovine;
(b) identifying a motor impairment (MI) haplotype in the genetic data, wherein the MI haplotype comprises:
   (i) one or more of the alleles in the shaded portion of FIG. 5, with the shaded portion of FIG. 5 corresponding to 78732954 to 80748266 bp of bovine chromosome 16; and/or
   (ii) a T on the positive DNA strand at position 79613592 bp on chromosome 16; and
(c) selecting or removing the bovine as breeding stock based on the identification of (b).

In some embodiments, the bovine is a heifer, cow, embryo or fetus. In some embodiments, the bovine is a bull. In some embodiments, the bovine is a Holstein. In some embodiments, the bovine is a cross of Holstein with another breed. In some embodiments, the bovine is phenotypically normal with respect to motor impairment. In some embodiments, the sample comprises one or more of semen, oocytes, tissue, cells, or blood.

In some embodiments, the genetic data comprises the DNA sequence of at least one chromosome 16 of the bovine at position 78732954 to 80748266 bp, or a fragment thereof. In some embodiments, the genetic data comprises the DNA sequence of both chromosomes 16 of the bovine at position 78732954 to 80748266 bp, or fragments thereof. In some embodiments, the MI haplotype comprises one or more SNPs at one or more positions 78732954 to 80748266 bp, or a fragment thereof, of bovine chromosome 16 associated with an MI phenotype, and genetic variants in linkage disequilibrium (LD) with those SNPs.

Also disclosed is genetic data that comprises RNA derived from chromosome 16 of a bovine at position 78732954 to 80748266 bp, or a fragment thereof. In some instances, the RNA is derived from the calcium voltage-gated channel subunit alpha1 S gene (CACNA1S).

In some embodiments, generating genetic data comprises sequencing the chromosomal DNA, such as by next generation sequencing. In some embodiments, generating genetic data comprises an amplification step, such as a polymerase chain reaction. In some embodiments, generating genetic data comprises a DNA microarray, beadchip, or oligonucleotide binding.

The present invention further provides a method comprising: detecting a homozygous negative for an MI haplotype in a genomic DNA sample from a bull, and identifying the bull as suitable for selective breeding to a female bovine, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

Also disclosed is using semen from the bull for fertilizing a female animal. In some instances, the female animal is *in vitro* fertilized. In some embodiments, the female is also homozygous negative for the MI haplotype. In some embodiments, the bull is a Holstein, Holstein crossbred, or bull of another breed with Holstein ancestry, and wherein the female animal is a Holstein, Holstein crossbred, or cow or heifer of another breed with Holstein ancestry.

The present invention further provides an *in vitro* method, the method comprising: detecting a homozygous negative MI haplotype in a sample comprising genomic DNA sample obtained from a female bovine or an embryo derived from the female bovine, thereby identifying oocytes from the female as suitable for in-vitro embryo production, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

Also disclosed is using oocytes from the female for *in-vitro* embryo production. In some embodiments, the female bovine is a Holstein, Holstein crossbred, or cow or heifer of another breed with Holstein ancestry.

The present invention further provides a method comprising: ascertaining the MI haplotype on a genomic DNA sample from a bull and a cow and identifying a homozygous negative female for the MI haplotype to mate to a bull that is positive for the MI haplotype, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

The present invention further provides a method comprising: ascertaining the MI haplotype from a genomic DNA sample obtained from a bull and identifying a homozygous negative bull for the MI haplotype and a female that is positive for the MI haplotype for corrective breeding, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

Also disclosed herein are compositions, kits, and systems. In particular, the present invention also provides a composition, kit, or system comprising: at least one nucleic acid molecule, wherein the nucleic acid molecule comprises SEQ ID NO: 1, SEQ ID NO: 2, or a fragment of SEQ ID NO: 1 or SEQ ID NO: 2, wherein the fragment includes the MI SNP and wherein the fragment includes at least 10 nucleotides 5' or at least 10 nucleotides 3' of the MI SNP position. In some embodiments, the nucleic acid molecule is linked/attached to a solid support. In some embodiments, the compositions, kits, and systems further comprise an enzyme having polymerase activity, such as Taq polymerase. In some embodiments, the nucleic acid molecule comprises one or more of a detectable marker, a linker (e.g., to attach to a solid support), and/or a non-natural nucleotide base.

These and other embodiments, aspects, advantages, and features of the present invention will be set forth in part in the description which follows and will become apparent to those skilled in the art by reference to the following description of the invention and referenced drawings or by practice of the invention. The accompanying drawings illustrate one or more implementations, and these implementations do not necessarily represent the full scope of the invention.

### BRIEF DESCRIPTION FO THE DRAWINGS

The present invention will be better understood and features, aspects, and advantages other than those set forth above will become apparent when consideration is given to the following detailed description thereof. Such detailed description refers to the following drawings, where:

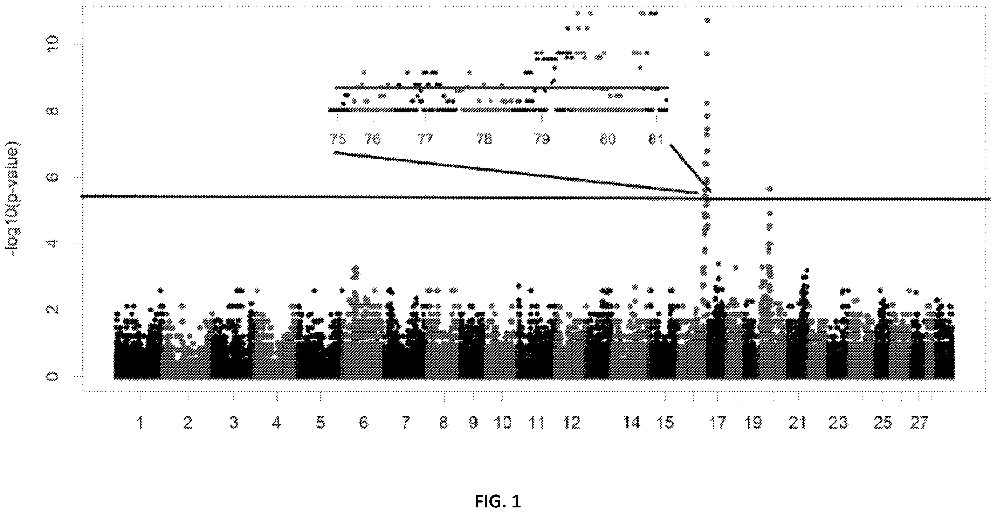

FIG. 1. Manhattan plot of significance levels for association of motor impairment with 101,917 DNA markers based on a dominance model with a significant region on chromosome 16 magnified; markers that exceed the genome wide false discovery rate significance level are above the solid black (main) and blue (inset) lines.

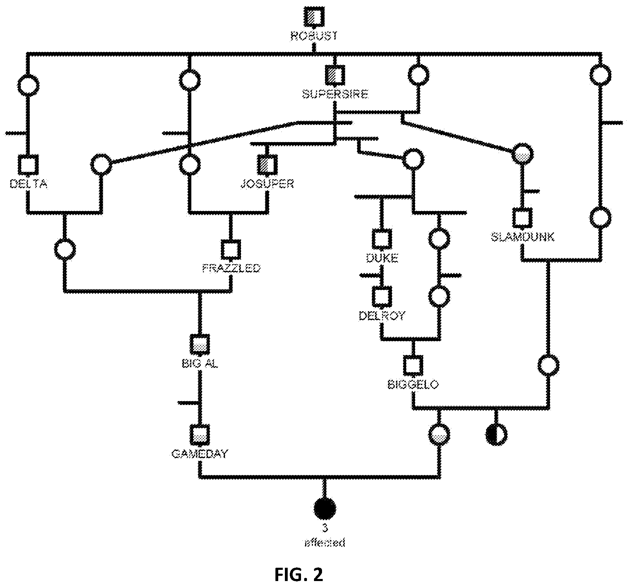

FIG. 2. Possible inheritance pathways of motor deficiency for the pedigree with the most pathways from the likely common ancestor (Robust) to 3 affected calves; genotypes are represented by half vertical-filled grey (probable carrier based on imputed genotypes), half vertical-filled black (carrier based on genotyping), half horizontal-filled grey (probable carrier based on offspring genotype), open with black outline (uncertain status), or filled (affected offspring) squares (male) or circles (female).

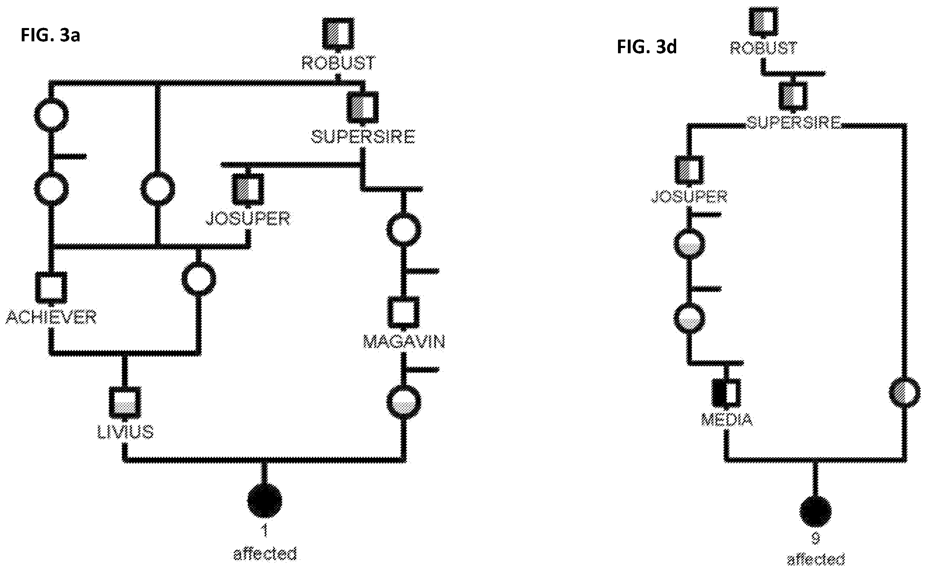

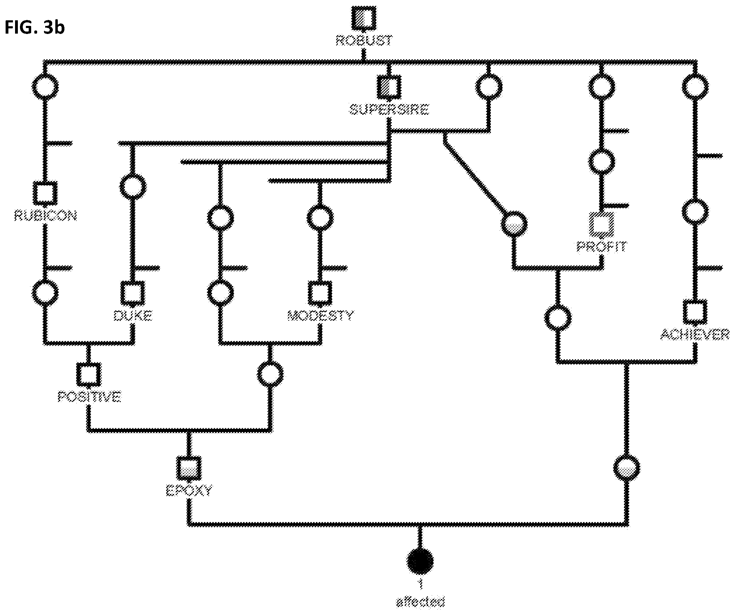

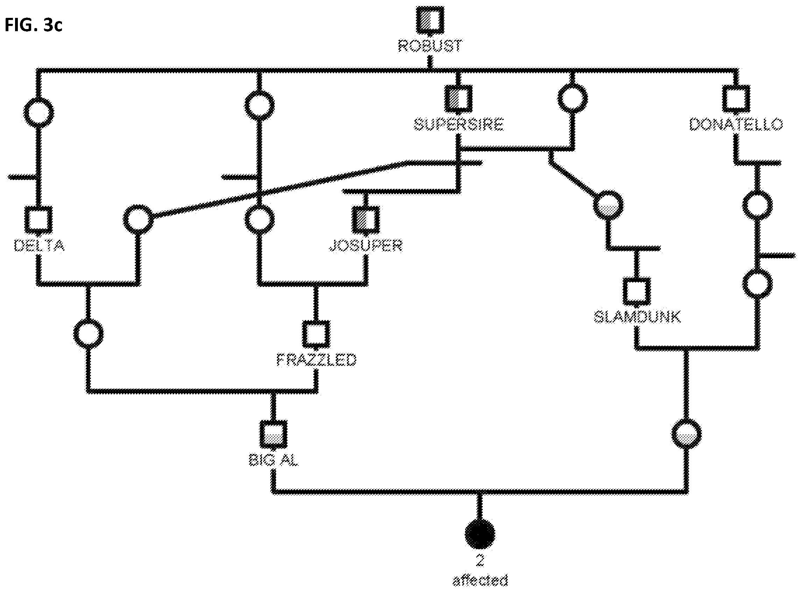

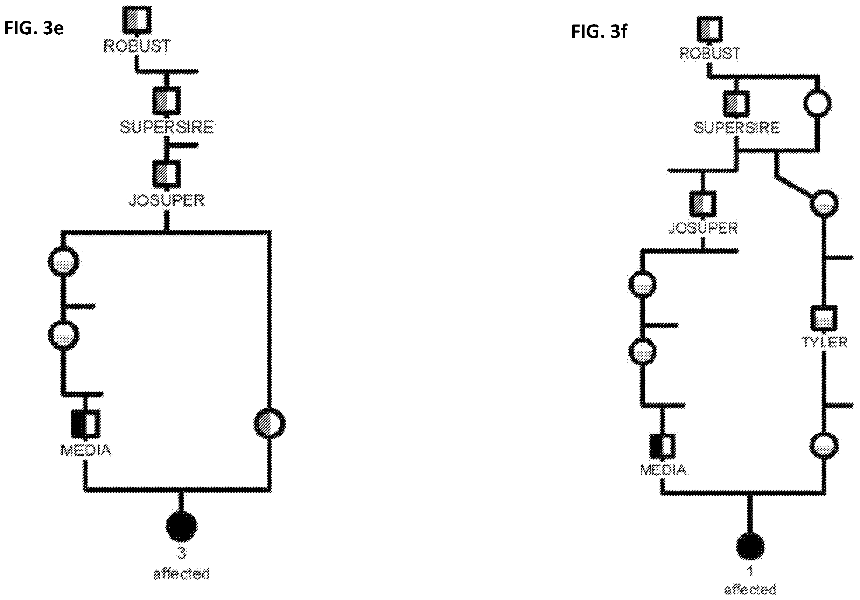

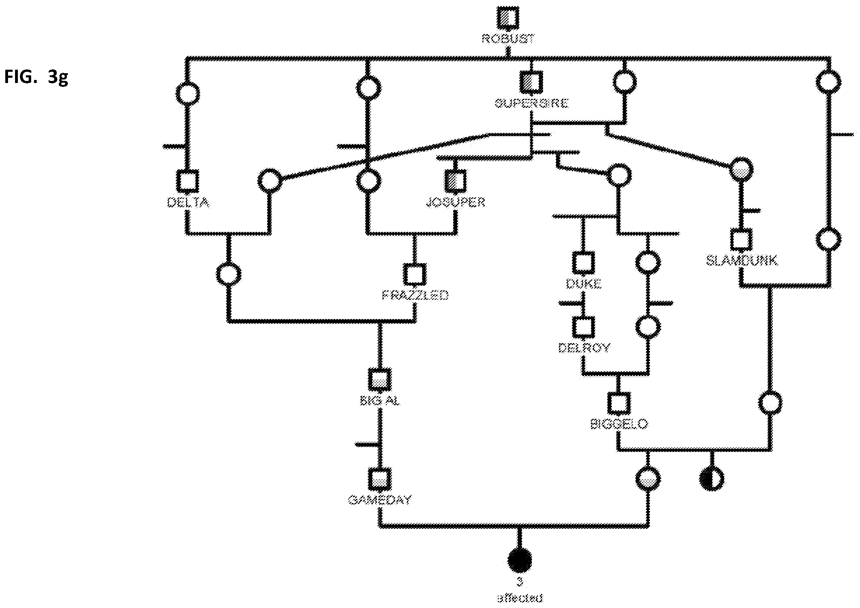

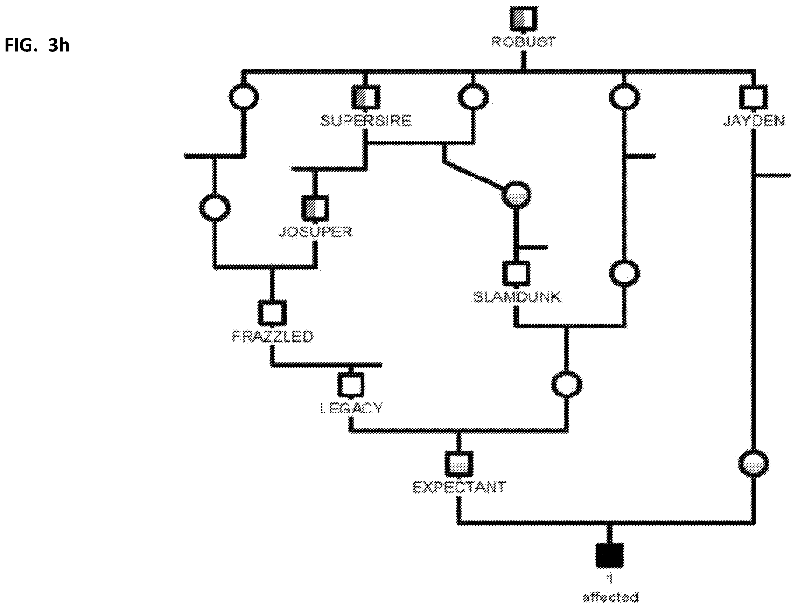

FIG. 3a, FIG. 3b, FIG. 3c FIG. 3d, FIG. 3e, FIG. 3f, FIG. 3g, FIG. 3h. Inheritance pathways of motor deficiency from the likely common ancestor (ROBUST) in 8 families of affected calves from farms 1 (a), 2 (b, c), 3 (d, e, f), and 4 (g, h); genotypes are represented by half vertical-filled grey (probable carrier based on imputed1 genotypes), half vertical-filled black (carrier based on genotyping), half horizontal-filled grey (probable carrier based on offspring genotype), open with black outline (uncertain status), open with green outline (probable non-carrier based on imputed genotype), or filled (affected offspring) squares (male) or circles (female). In Figure 3h, imputed genotypes were derived using findhapf90 using the genotype from affected calves, controls, and a population of 7622 Holsteins with genotype densities ranging from 3K to 150K.

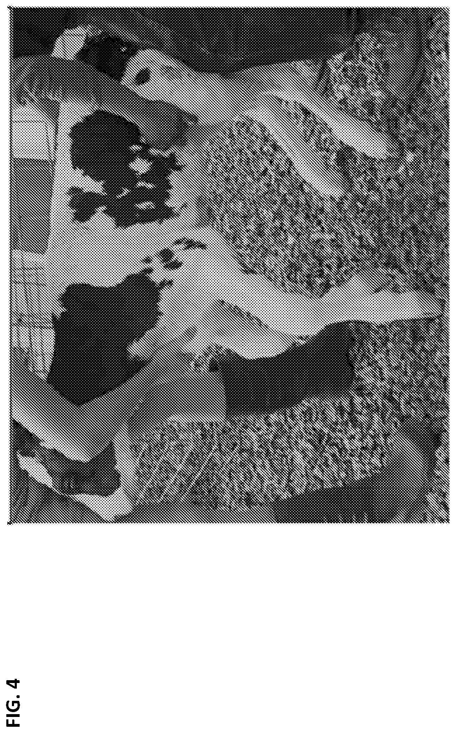

FIG. 4. A photograph of a calf unable to stand, exhibiting the MI (recumbent) phenotype.
FIG. 5. DNA markers, their location, the alternate allele forward designations (Allele1_F and Allele2 _F), alternate allele top designations (Allele1_Top and Allele2_Top), and alternate allele A and B designations (Allele1_AB and Allele2_AB) present in the genotyped population; number of affected calves with the AA (nAA), AB (nAB), and nBB (nBB) genotypes; and unadjusted p-values for the allelic model (p_Allelic), false discovery rate p-values for the allelic model (FDRp_Allelic), and false discovery rate p-values for the dominance model (FDRp_Dom) for the end of chromosome 16. The shaded region (starting at CHR 16 BP 78732954 and continuing to CHR 16 BP 8074266) represents the shared homozygous region on chromosome 16 (the haplotype region).

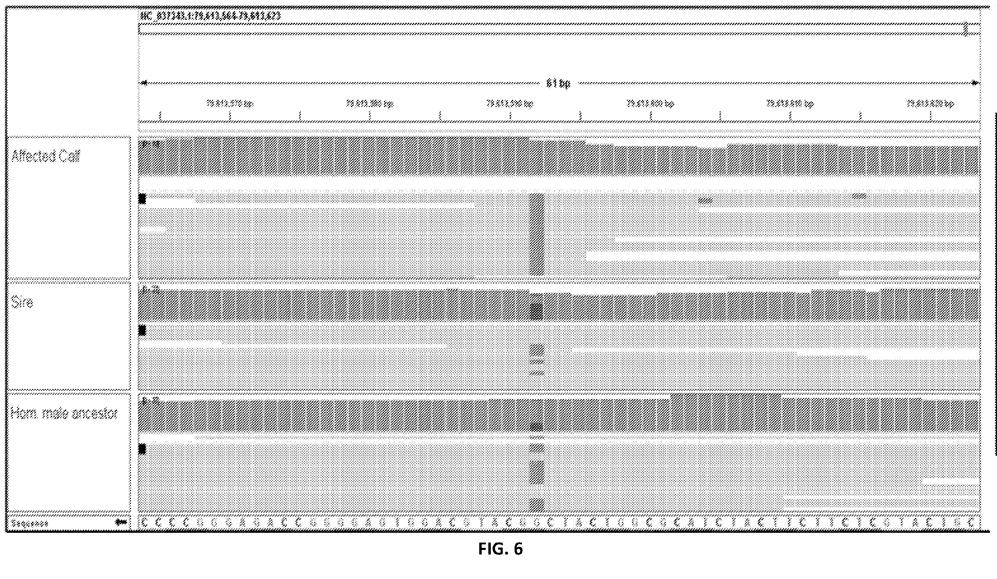

FIG. 6. Sequence alignments for the recumbency mutation for the affected calf, sire, and male ancestor at bp 79613592. The top panel for each animal represents the sequence depth with red indicating the mutation and blue indicating the reference genotype, whereas the bottom panels indicate individual reads with red indicating mutant genotypes and grey the reference genotype. The affected calf reads are homozygous and the sire and male ancestor are heterozygous.

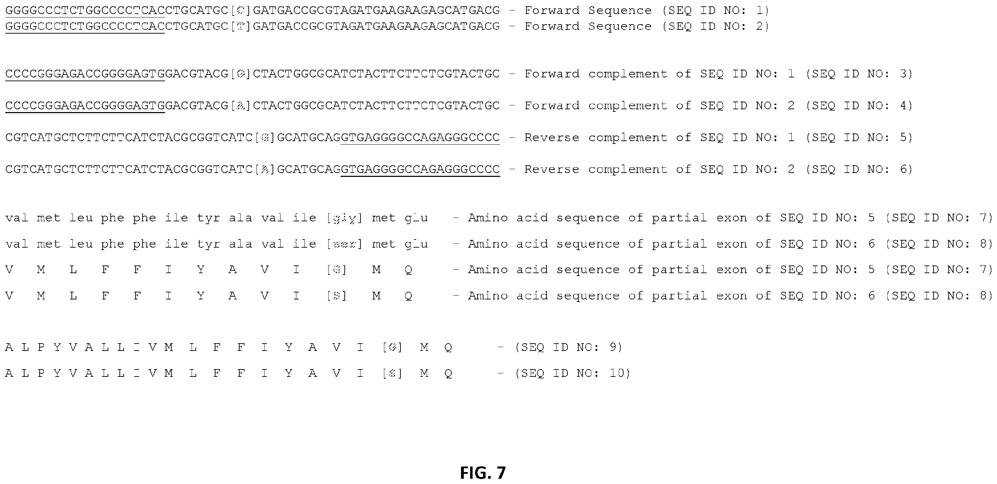

FIG. 7. Nucleotide and amino acid sequences flanking either side of, and including, the MI SNP. The underlined sequence corresponds to intron. SEQ ID NOs: 9 and 10 are also shown in Table 4.

### DETAILED DESCRIPTION

The present invention is described herein using several definitions, as set forth below and throughout the application.

Unless otherwise specified or indicated by context, the terms "a", "an", and "the" mean "one or more." Thus, the references "a", "an", and "the" are generally inclusive of the plurals of the respective terms. For example, reference to "a SNP", "a method", or "a trait" includes a plurality of such "SNPs", "methods", or "traits." Reference herein, for example to "an association" includes a plurality of such associations, whereas reference to "chromosomes" includes a single chromosome where such' interpretation is not precluded from the context. Similarly, the words "comprise", "comprises", and "comprising" are to be interpreted inclusively rather than exclusively. Likewise the terms "include", "including" and "or" should all be construed to be inclusive, unless such a construction is clearly prohibited from the context. Where used herein the term "examples," particularly when followed by a listing of terms is merely exemplary and illustrative, and should not be deemed to be exclusive or comprehensive.

As used herein, "about," "approximately," "substantially," and "significantly" will be understood by persons of ordinary skill in the art and will vary to some extent on the context in which they are used. If there are uses of these terms which are not clear to persons of ordinary skill in the art given the context in which they are used, "about" and "approximately" will mean plus or minus ≤ 10% of the particular term and "substantially" and "significantly" will mean plus or minus > 10% of the particular term.

As used throughout, ranges herein are stated in shorthand, so as to avoid having to set out at length and describe each and every value within the range. Any appropriate value within the range can be selected, where appropriate, as the upper value, lower value, or the terminus of the range. For example, a range of 0.1-1.0 represents the terminal values or 0.1 and 1.0, as well as the intermediate values of 0.2, 0.3, 0.4, 0.5, 0.6, 0.7, 0.8, 0.9, and all intermediate ranges encompassed within 0.1-1.0, such as 0.2-0.5, 0.2-0.8, 0.7-1.0, and so on.

As used herein, the terms "bovine" and "cattle" generally refer to members of the genus *Bos,* and include, without limitation, *Bos taurus* and *Bos indicus* (also termed *Bos taurus indicus).* The disclosed compositions, systems, methods, kits, and platforms are not intended to be limited to members of the *Bos* genus, and may be used with other members of the family *Bovidae.* By way of example, but not by way of limitation, in some embodiments, the disclosed compositions, systems, methods, kits, and platforms are used to detect an MI haplotype in a domestic bovine, such as a Holstein, Holstein crossbreed, or animal of another breed with Holstein ancestry. In the cattle industry, most breeds have a "grade-up" program, resulting, for example, in animals having Holstein genes present in a different breed and vice-versa.

The term "genotype" as used herein refers to the identity of the alleles present in an individual or a sample. In the context of the present invention, a genotype preferably refers to the description of the polymorphic alleles present in an individual or a sample. The term "genotyping" a sample or an individual for a polymorphic marker refers to determining the specific allele or the specific nucleotide carried by an individual at a polymorphic site.

As used herein, "locus", or plural "loci", refers to a physical site or location of a specific gene or marker on a chromosome. An exemplary locus comprises position 78732954 to 80748266 bp on chromosome 16 of a bovine, such as a Holstein. In some embodiments a locus is a single base pair (bp, or BP), such as, for example, bp 79613592 on chromosome 16 of a Holstein.

As used herein, "linkage disequilibrium" (or "LD") refers to the allelic association between specific alleles at two or more neighboring loci in the genome, e.g., within a population. LD can be determined for a single marker or locus, or multiple markers. Stated another way, LD is the correlation between nearby variant such that the alleles at neighboring polymorphisms (observed on the same chromosome) are associated within a population more often than if they were unlinked.

As used herein, "allele" refers to one or more alternative forms of a particular nucleic acid sequence, where the differences may include, without limitation, one or more single nucleotide polymorphisms (SNPs), an insertion, inversion, or deletion. The sequence may or may not be within a gene, and may be within a coding region or noncoding region of a gene, and may, for example, be within a promoter or regulatory regions, an exon, or an intron of a particular gene. By way of example, the sequence of the positive DNA strand from bp 79613564 to 79613623 of Holstein, with the alleles (in this case a SNP) indicated as [C] or [T] is: GGGGCCCTCTGGCCCCTCACCTGCATGC[C/T]GATGACCGCGTAGATGAAGAAGAGC ATGACG (SEQ ID NO: 1 and 2). Underlined sequence indicates intron.

The term "haplotype" refers to a combination of alleles or DNA markers on one chromosome. At the DNA level, haplotype refers to a sequence of nucleotides found at two or more polymorphic sites in a locus on a single chromosome. There are multiple haplotypes over a given chromosome region within a breeding population. As used herein, haplotype includes a full-haplotype and/or a sub-haplotype. Full-haplotype is the 5' to 3' sequence of nucleotides found at all polymorphic sites examined in a locus on a single chromosome from a single individual, while sub-haplotype refers to the 5' to 3' sequence of nucleotides seen at a subset of the polymorphic sites examined in a locus on a single chromosome from a single individual. Relatedly, the term "haplotype pair" refers to the two haplotypes found for a locus in a single individual. "Haplotyping" is a term for a process for determining one or more haplotypes in an individual and includes use of family pedigrees, molecular techniques and/or statistical inference. By way of example but not by way of limitation, the MI haplotype is shown by the shaded region of Figure 5, encompassing 78732954 to 80748266 bp of bovine chromosome 16.

"Quantitative trait locus," (or "QTL"), as used herein is a genomic sequence that is associated with a particular phenotypic trait. Multiple QTL may be identified for a particular trait, and they are frequently found on different chromosomes. The number of QTLs that associate significantly with a particular phenotypic trait may provide an indication of the genetic architecture of a trait, the number of genes that affect the trait, or the extent of the effect of one or more of those genes. One or more QTL that significantly associates with a trait may be candidate genes underlying that trait, which can be sequenced and identified. The significance of the degree of association of a given QTL with a particular trait can be assessed statistically, e.g. through QTL mapping of the alleles that occur in a locus and the phenotypes that they produce. Statistical analysis is preferred to demonstrate whether an observed association with a trait is significant. The presence of a QTL, and its location identify a particular region of the genome as potentially containing a gene that is associated, directly (e.g., structurally) or indirectly (e.g., regulatory) with the trait being analyzed. The probability of association can be plotted for various markers associated with the trait spaced across a chromosome, or throughout the genome. A QTL may be present in a haplotype that is associated with a phenotype.

A "polynucleotide" includes single-stranded or a multi-stranded nucleic acid molecules comprising two or more sequential bases, including any single strand or parallel and anti-parallel strands of a multi-stranded nucleic acid. Polynucleotide may be of any length, and thus, include very large nucleic acids, as well as short ones, such as oligonucleotides.

The term "oligonucleotide" typically refers to short polynucleotides, generally no greater than about 50 nucleotides. It will be understood that if a nucleotide sequence is denoted represented by a DNA sequence (i.e., A, T, G, C), the corresponding RNA sequence (i.e., A, U, G, C, wherein "U" replaces "T") is also included unless otherwise specified, or unless context and common sense make clear the distinction.

As used herein, the term "motor impairment," used interchangeably with the term "recumbent" or "recumbency" refers to a condition in which an animal is unable to coordinate, control, or respond normally with respect to voluntary muscle movement. By way of example, motor impairment may include the following traits noted, for example, in a calf: the inability to stand; the inability to stand without assistance; the inability to stand for the first 24 hours after birth; losing the ability to stand; the inability to stand, followed by recovery of the ability to stand, followed by poor growth and health. Motor impairment as used herein is not accompanied by or due to abnormal nutrition, injury, or metabolite levels. Figure 4 provides an example of recumbency in a Holstein calf. Recumbent animals exhibit normal mention, appetite, and behavior, despite an inability to rise or remain standing. In addition, recumbency as defined herein is not due to abnormalities such as anemia, cholesterol, selenium or vitamin deficiency, and disease or infection (e.g., such as neospora, bovine viral diarrhea, rabies and listeria). Recumbent animals as disclosed herein exhibit unremarkable histologic sections of the central nervous system, peripheral nerves and muscle tissue.

As used herein, the term "motor impairment haplotype" (MI haplotype) refers to the haplotype of an animal, such as a Holstein bovine, at position 78732954 to 80748266 bp on chromosome 16. A positive MI haplotype is indicative of an animal that exhibits, or is likely to exhibit motor impairment as defined above, or that may pass motor impairments to its offspring. A negative MI haplotype is indicative of an animal that is unlikely to exhibit motor impairment, and that is unlikely to pass motor impairments to its offspring. By way of example, a positive MI haplotype is represented by Figure 5; the alleles presented in the shaded region of FIG. 5 (78732954 to 80748266 bp of chromosome 16) are illustrative of the MI haplotype. In some embodiments, the shaded alleles of FIG. 5 are evaluated for MI haplotype determination. Conversely, a negative MI haplotype, indicating an animal without MI, is identified when the animal inherits an alternative haplotype over the chromosome region which is indicated by a different combination of DNA markers.

While the MI phenotype is associated with the MI haplotype, it is also associated with a SNP [C/T]: The sequence of the positive DNA strand from bp 79613564 to 79613623 with the SNP indicated as [C] or [T] (79613592 bp) is shown below:

GGGGCCCTCTGGCCCCTCACCTGCATGC[C/T]GATGACCGCGTAGATGAAG
AAGAGCATGACG (SEQ ID NO: 1 and 2).

Underlined sequence represents intron.

The Forward Strand, Reverse Strand, Reverse Complement and amino acid sequence of this region is shown in FIG. 7. As used herein, the [C] or [T] as displayed in SEQ ID NO: 1 or SEQ ID NO: 2, respectively, (or its complement or reverse complement) will be termed the MI SNP.

The MI SNP is present in the calcium voltage-gated channel subunit alpha1 S gene (CACNA1S) of Holstein cattle. The missense mutation alters a GGC codon to AGC which facilitates a glycine to serine amino acid substitution. The complete protein sequence of CACNA1S is shown below.

The amino acid sequence of CACNA1S (XP_024832342.1 CACNA1S [organism=Bos taurus] [GeneID=100337204] [isoform=X1]) is shown below (SEQ ID NO: 11 and 12):

MEPAPAQDEGLRRKQAKKPVPEVLPRPPRALFCLTLQNPVRKACISIVEWKPF
ETIILLTIFANCVALAVYLPMPEDDNNRLNLGLEKLEYFFLIVFSIEAAMKIIAYGFLFHQD
AYLRSGWNVLDFIIVFLGVFTVILEQVNLIQSNTAPMSSKGAGLDVKALRAFRVLRPLRL
VSGVPSLQVVLNSIFKAMLPLFHIALLVLFMVVIYAIIGLELFKGKMHKTCYFIGTDIVAT
VENEKPSPCARTGSGRPCTISGSECRGGWPGPNHGITHFDNFGFSMLTVYQCITMEGWT
DVLYWVNDAIGNEWPWIYFVTLILLGSFFILNLVLGVLSGEFTKEREKAKSRGTFQKLRE
KQQLEEDLRGYLSWITQGEVMDVDDLREGKLALDEGGSDTESLYEIAGLNKVIQFIRHW
RQWNRVFRWKCHDVVKSRVFYWLVILIVALNTLSIASEHHHQPLWLTHLQDVANRVLL
ALFTVEMLMKMYGLGLRQYFMSIFNRFDCFVVCSGLLEILLVESGAMSPLGISVLRCIRL
LRIFKITKYWTSLSNLVASLLNSIRSIASLLLLLFLFIVIFALLGMQLFGGRYDFEDTEVRR
SNFDSFPQALISVFQVLTGEDWNSVMYNGIMAYGGPSYPGVLVCIYFIILFVCGNYILLN
VFLAIAVDNLAEAESLTSAQKAKAEERRRRKMSKGLPDKSEEEKVTVAKKLEQKPKGE
GIPTTAKLKIDEFESNVNEVKDPYPSADFPGDDEEDEPEVPISPRPRPLAELQLKEKAVPIP
EASAFFIFSPTNKIRVLCHRIVNATWFTNFILLFILLSSAALAAEDPLRAESVRNQILGYFDI
GFTSVFTVEIVLKMTTYGAFLHKGSFCRNYFNILDLVVVAVSLISMGLESSTISVVKILRV
LRVLRPLRAINRAKGLKHVVQCVFVAIRTIGNIVLVTTLLQFMFACIGVQLFKGKFFSCN
DLSKMTEEECRGYYYVYKDGDPTQIEVRPRQWVHNAFHFDNVLSAMMSLFTVSTFEG
WPELLYKAIDSHEEDKGPVYNHRVEMAIFFIIYIILIAFFMMNIFVGFVIVTFQEQGETEYK
NCELDKNQRQCVQYALKARPLRCYIPKNPYQYRVWYIVTSSYFEYLMFALIMLNTICLG
MQHYNQSEQMNHISDILNVAFTIIFTLEMVLKLMAFKARGYFGDPWNVFDFLIVIGSIID
VILSEIDTFLASSGGLYCLGGGCGNVDPDESARISSAFFRLFRVMRLIKLLSRAEGVRTLL
WTFIKSFQALPYVALLIVMLFFIYAVI[G/S]MQMFGKIALVDGTQINRNNNFQTFPQAVL
LLFRCATGEAWQEILLACSYGQLCDPESDYAPGEEYTCGTDFAYYYFLSFYMLCAFLIIN
LFVAVIMDNFDYLTRDWSILGPHHLDEFKAIWAEYDPEATGRIKHLDVVTLLRRIQPPLG
FGKFCPHRVACKRLVGMNMPLNSDGTVTFNATLFALVRTALKIKTEGNFEQANEELRAI
IKKVWKRTSMKLLDQVIPPIGDDEVTVGKFYATFLIQEHFRKFMRRQEEYYGYRPKKDT
VQIQAGLRTIEEEAAPEIRRTISGDLMAEEELERAMVEAAMEEGIFRRTGGLFGQVDSFL
ERTNSLAPHMANQRPLQFTEIEMEEMESPVFLEDFPQRPSAHPLARANTNNANANVASG
NGNHGNSPAFPSVHYEGELAGETETPATRGGLFGQPRGALGPHSKSRVEWPQGQLPRR
RTPRAQPAASCQHAGAESQVPEERSPTPGSLPGEAPPGSRTEDGTPGGPAPAMALLIRE

ALVRGGLDSLAADANFIMATGQALAEACRMEPREVEVAAAELLQGREALEGVPGARG
CPGLGSSLGSLDLREGSQETLIPRRP

The nucleic acid sequence encoding the CACNA1S amino acid sequences (XM_024976574.1 PREDICTED: Bos taurus calcium voltage-gated channel subunit alpha1 S (CACNA1S), transcript variant X1, mRNA) is shown below (SEQ ID NO: 13 and 14):

GCTCAGCCGCCCAGTCCCGGAGGCGGGGCGCTGGACGCGGGAGGAG
AGGACAGCGGTAGGGGAGCAGGAAAGGCCGAGGCCGCGGAGCCATGGAGCCGGC
CCCGGCACAGGACGAGGGCCTGCGGAGGAAGCAGGCCAAGAAGCCCGTCCCGGAG
GTCCTGCCCAGGCCACCCCGGGCCTTGTTCTGCTTGACCCTCCAGAACCCCGTGCGG
AAAGCCTGCATCAGCATCGTCGAGTGGAAACCCTTCGAGACCATCATCTTACTCACC
ATCTTTGCCAACTGCGTGGCCCTGGCCGTGTACCTGCCCATGCCGGAGGACGACAAC
AACAGACTGAACCTCGGCCTGGAGAAGCTGGAATACTTCTTCCTCATCGTCTTCTCC
ATCGAGGCCGCCATGAAGATCATCGCCTACGGCTTCCTGTTCCACCAGGATGCCTAC
CTGCGCAGCGGCTGGAACGTGCTGGATTTCATCATCGTCTTCCTGGGGGTCTTCACC
GTGATCCTGGAGCAGGTCAACCTCATCCAGAGCAACACGGCCCCGATGAGCAGCAA
AGGCGCGGGCCTGGACGTCAAGGCACTGCGGGCCTTCCGCGTGCTCCGTCCGCTCCG
GCTGGTGTCCGGGGTGCCCAGCCTGCAGGTGGTCCTCAACTCCATCTTCAAGGCCAT
GCTGCCGCTCTTCCACATCGCCCTGCTGGTGCTCTTCATGGTCGTCATCTACGCCATC
ATCGGCCTCGAGCTCTTCAAGGGCAAGATGCACAAGACCTGCTACTTCATCGGCACA
GACATCGTGGCCACGGTGGAGAACGAGAAGCCGTCCCCCTGCGCCCGGACGGCTC
CGGCCGGCCCTGCACCATCAGCGGCAGCGAGTGCCGTGGCGGGTGGCCCGGCCCCA
ACCACGGCATCACGCACTTCGACAACTTCGGCTTCTCCATGCTCACCGTCTACCAGT
GCATCACCATGGAGGGCTGGACCGACGTCCTCTACTGGGTGAATGATGCTATCGGG
AACGAGTGGCCCTGGATCTACTTTGTCACCCTCATCCTGCTGGGCTCCTTCTTCATCC
TCAACCTGGTGCTGGGCGTCCTGAGCGGGGAATTCACCAAGGAGCGGGAGAAGGCC
AAGTCCAGGGGGACCTTCCAGAAGCTGCGGGAGAAGCAGCAGCTGGAGGAGGACC
TCCGGGGCTACCTGAGCTGGATCACGCAGGGCGAGGTCATGGACGTGGATGACTTG
AGAGAAGGGAAGCTGGCTCTGGATGAAGGCGGCTCCGACACGGAGAGCCTGTACGA
AATCGCGGGCCTGAACAAAGTTATCCAGTTCATCCGACACTGGAGGCAGTGGAACC

GTGTCTTCCGCTGGAAGTGCCACGATGTGGTGAAGTCCAGGGTGTTCTACTGGCTGG
TCATCCTGATCGTCGCTCTCAACACACTGTCGATCGCCTCAGAGCACCACCACCAGC
CTCTCTGGTTGACCCACCTGCAAGACGTGGCCAACCGGGTGCTGCTGGCCCTGTTCA
CCGTGGAGATGCTGATGAAGATGTACGGGCTGGGCCTGCGCCAGTACTTCATGTCCA
TCTTCAACCGCTTCGACTGCTTCGTGGTGTGCAGCGGCCTCCTGGAGATCCTGCTGGT
GGAGTCGGGTGCCATGTCGCCCCTGGGCATCTCGGTGCTGCGCTGCATCCGTCTGCT
GCGGATCTTCAAGATCACCAAGTACTGGACGTCGCTGAGCAACCTGGTGGCGTCGCT
GCTCAACTCCATCCGCTCCATCGCCTCGCTGCTGCTGCTCTTCCTGTTCATCGTC
ATCTTCGCCCTGCTGGGCATGCAGCTCTTCGGGGGCAGGTACGACTTCGAGGACACC
GAGGTGCGGCGTAGCAACTTCGACAGCTTCCCACAGGCCCTCATCAGCGTCTTCCAG
GTGCTGACAGGAGAGGACTGGAACTCCGTGATGTACAACGGGATCATGGCCTACGG
CGGCCCATCCTACCCCGGGGTGCTCGTCTGCATTTACTTCATCATCCTGTTTGTTTGC
GGCAACTACATCCTGCTCAACGTCTTCCTGGCCATCGCTGTGGACAACCTGGCAGAG
GCCGAGAGCCTGACTTCTGCCCAGAAGGCCAAGGCTGAAGAGCGGAGACGCAGGA
AGATGTCCAAGGGTCTCCCGGACAAGTCAGAGGAGAAGGTGACGGTGGCCAA
GAAGCTGGAGCAGAAACCCAAGGGGGAAGGCATCCCCACCACCGCCAAGCTGAAG
ATCGACGAGTTTGAATCCAACGTCAACGAGGTGAAGGACCCCTACCCCTCAGCCGA
CTTCCCAGGGGACGACGAGGAAGACGAGCCCGAGGTCCCAATAAGCCCCCGGCCCC
GTCCGCTGGCTGAGCTGCAGCTGAAGGAGAAGGCGGTGCCCATTCCAGAAGCCAGT
GCCTTCTTCATCTTCAGCCCCACCAACAAGATCCGCGTCCTGTGCCACCGCATTGTC
AATGCCACCTGGTTCACTAACTTCATCCTGCTCTTCATCCTGCTCAGCAGTGCCGCCT
TGGCCGCCGAGGACCCCCTCCGGGCTGAGTCCGTGAGGAACCAGATCCTCGGGTAT
TTTGACATCGGGTTCACCTCTGTCTTCACTGTGGAGATTGTCCTCAAGATGACCACCT
ACGGGGCCTTCCTGCACAAGGGCTCCTTCTGCCGCAACTACTTCAACATCCTGGACC
TGGTGGTGGTGGCCGTGTCCCTCATCTCCATGGGACTCGAGTCCAGCACCATCTCCG
TGGTGAAGATCCTGAGGGTCCTGAGGGTGCTCCGACCGCTCCGGGCCATCAACAGA
GCCAAAGGCCTGAAGCACGTGGTACAGTGTGTGTTCGTGGCCATCCGCACCATCGG
GAACATCGTCCTGGTCACCACCCTCCTGCAGTTCATGTTCGCTTGCATCGGCGTCCA
GCTCTTCAAGGGGAAGTTCTTCAGCTGCAATGACTTGTCCAAGATGACGGAGGAGG
AGTGCAGGGGCTACTACTATGTGTACAAAGATGGAGACCCCACACAGATAGAGGTG

CGTCCCCGCCAGTGGGTGCACAACGCCTTCCACTTCGACAACGTGCTTTCGGCCATG
ATGTCCCTCTTCACCGTGTCCACCTTCGAGGATGGCCTGAGCTGCTGTACAAGGCC
ATTGACTCTCATGAGGAGGACAAGGGCCCCGTGTACAACCACCGCGTGGAGATGGC
CATTTTCTTCATCATCTACATCATCCTTATCGCCTTCTTCATGATGAACATCTTCGTGG
GCTTTGTCATCGTCACCTTCCAGGAGCAGGGGGAGACTGAGTACAAGAACTGTGAG
CTGGACAAGAACCAGCGCCAGTGCGTGCAGTACGCCCTGAAGGCCCGGCCCCTGAG
GTGCTACATCCCCAAGAACCCCTACCAGTACCGGGTGTGGTACATCGTCACCTCCTC
CTACTTCGAGTACCTGATGTTCGCCCTCATCATGCTCAACACCATCTGCCTGGGCAT
GCAGCACTACAACCAGTCGGAGCAAATGAACCACATCTCTGACATCCTCAACGTAG
CCTTCACCATCATCTTCACACTGGAGATGGTCCTCAAGCTCATGGCGTTCAAGGCCA
GGGGCTATTTTGGGGACCCCTGGAACGTGTTTGACTTCTGATTGTCATCGGCAGCA
TCATCGACGTCATCCTCAGTGAGATCGACACTTTCCTGGCCTCCAGCGGGGGACTGT
ATTGCCTGGGTGGCGGCTGCGGGAACGTTGACCCGGACGAGAGCGCCCGCATCTCC
AGCGCCTTCTTCCGCCTGTTCCGTGTCATGAGGCTGATCAAGCTGCTGAGCCGCGCG
GAGGGCGTGCGCACGCTGCTCTGGACTTTCATCAAGTCCTTCCAGGCCCTGCCCTAC
GTGGCTCTGCTCATCGTCATGCTCTTCTTCATCTACGCGGTCATC[G/A]GCATGCAGA
TGTTCGGGAAGATCGCCTTGGTGGACGGGACCCAGATCAACCGGAACAACAACTTC
CAGACCTTCCCCCAGGCGGTGCTGCTGCTCTTCAGGTGCGCCACGGGGGAGGCGTG
GCAGGAGATCCTGCTGGCCTGCAGCTACGGCCAGCTGTGTGACCCCGAGTCGGACT
ACGCCCCCGGGGAGGAGTACACCTGTGGCACGGACTTCGCCTACTACTACTTCCTCA
GCTTCTACATGCTGTGCGCCTTCCTGATCATCAACCTCTTTGTGGCCGTCATCATGGA
CAACTTTGACTACCTGACACGGGACTGGTCCATCCTGGGCCCTCATCACCTGGATGA
GTTCAAGGCCATCTGGGCGGAGTACGACCCGGAGGCTACGGGCAGGATCAAGCACC
TGGACGTGGTGACCCTGCTGAGGAGGATCCAGCCCCCGCTGGGCTTCGGGAAGTTCT
GCCCCCACCGGGTAGCGTGTAAGCGGCTGGTGGGCATGAACATGCCCCTCAACAGT
GACGGCACGGTCACCTTCAACGCCACGCTCTTCGCCCTGGTGCGCACAGCTCTGAAG
ATCAAGACGGAAGGTAACTTCGAGCAGGCCAACGAGGAGCTGCGAGCCATCATCAA
GAAGGTCTGGAAGAGGACCAGCATGAAGCTCCTGGACCAGGTCATCCCTCCCATAG
GAGATGACGAGGTGACGGTGGGGAAGTTCTACGCCACGTTCCTCATCCAGGAGCAC
TTCCGGAAGTTCATGAGGCGCCAGGAGGAGTATTATGGGTACCGGCCCAAGAAGGA

CACCGTGCAGATCCAGGCTGGACTGAGGACCATCGAGGAAGAGGCAGCCCCTGAGA
TCCGCCGCACCATCTCGGGAGACCTGATGGCCGAAGAGGAGCTGGAGAGAGCCATG
GTGGAGGCCGCGATGGAGGAAGGGATCTTCCGGAGGACCGGCGGCCTGTTTGGCCA
GGTGGACAGCTTCCTGGAAAGGACCAACTCGTTGGCCCCGCACATGGCCAACCAGA
GACCCCTCCAGTTTACTGAAATTGAGATGGAGGAGATGGAATCGCCCGTCTTCCTGG
AGGACTTCCCTCAACGTCCGAGTGCCCACCCCCTTGCTCGTGCCAACACCAACAACG
CCAACGCCAACGTCGCCAGTGGCAACGGCAACCATGGCAACAGCCCAGCATTTCCC
AGTGTCCACTACGAAGGGGAGCTCGCAGGGGAGACAGAGACGCCTGCCACCAGAG
GAGGACTCTTCGGCCAGCCCCGGGGGGCCCTGGGGCCCCACAGCAAGTCCCGAGTA
GAGTGGCCGCAGGGACAGCTGCCCCGGAGAAGGACGCCCAGGGCCCAGGCCCCCG
CTGCCTCCTGCCAGCACGCGGGAGCAGAGTCCCAGGTGCCCGAGGAGAGGAGCCCC
ACCCCAGGGTCCCTGCCCGGGGAGGCGCCGCCCGGCAGTAGGACAGAGGACGGAA
CCCCTGGGGGTCCAGCTCCAGCCATGGCCCTGCTGATCCGCGAGGCTCTGGTTCGAG
GGGGCCTGGACAGCTTGGCGGCGGATGCTAACTTCATCATGGCCACGGGCCAGGCG
CTGGCAGAGGCCTGCCGGATGGAACCGCGGGAAGTGGAGGTGGCAGCCGCAGAGC
TTCTGCAGGGACGCGAGGCCCTGGAGGGCGTGCCTGGTGCCCGAGGGTGCCCAGGC
CTCGGGTCCTCCCTGGGCAGCCTCGACCTGCGTGAGGGCTCCCAGGAGACCCTGATT
CCCCGCAGGCCATGAAGGCCTCGGCGTCTGCGTGGGCCCAGGCTGGCGAGACCGAG
GGTGGGGGAGCTGAAGCGGGAGAGTTAGCCCGGCTCGGCAGCCTCGATGGACAGAG
GGGTGGGCTGGGGCGGGCAAGGGGGTCAGGAAACGCGTCCAAGGTCAGCGGGCAG
AAGGTGAAGTCACTGTCCTCACCAGCAAGCCGCGTGAGTGGACAGAGCTTCTGTGTC
GTTCAGAGGCCTGGTCAGTGCTGGCAGGACATTAAAGTCTCCAGGCCTGCGACAGG
GCACTGCTTGTC

The exon including the MI SNP and both flanking introns (CACNA1S/XM_024976575.1/XP_024832343.1 79612962 - 79614080) are shown in the nucleic acid sequence below (SEQ ID NO: 15 and 16). The underlined sequence corresponds to intron:

CTGCAGGCCACGAAAGGCTGGACTGTGGACCTGGACAGGTCTCTCACCGG
CCCCAGCTCCCCCGACACCCTCCACCTCAGGTCACACAAACCGGGCTACAGCTGACC
CCTGAGCAACGCCAGGGTATAGGTCTACAGTCAGCTCCCCGCCCTGTATCCACGGAT

TCAACCAACCATGAATCCCGTGGAGCCCTAGTACAGTTCCCTGAAAAATTCACGGAT
ACATGGACACCCGAAGTTCAAATCTGCCTGTTCAAGGCTCACTTATGTTTCCAAGGT
TGGGTGAAATTTTTCCCCTCAAGCCAAATTAATCTGGAGGGGACACTGTTCCTAA
CCAAGAGCTGACTTAGGGTCTCAATGTAGAGAGCAGAGTGCTTCAGGAGAGGGTAG
CTGAGACCACCAGAGCCTCCAACAGGGGCTTTGGAGACCAGTTCCGCCCTTGTCCAT
CTGCCCACAAGGTTCCCCTGGGCTGCCTCCTCCTGACTGATGCTGGGCCGGCTGGGA
ACCACCTCCTCCTCTAGGGCTGTGGCCAAGCCCCGGGCTGCCAGGATGCAGGAGGC
AGGCTGGTGAGAGGCCAGCCTGCAGGCCGGCCGGCCTCCCGGGGGCCCTCTGGCCC
CTCACCTGCATGC[C/T]GATGACCGCGTAGATGAAGAAGAGCATGACGATGAGCAG
AGCCACGTAGGGCAGGGCCTGCGGGCGAGGATGGCGGCTCAGGGTGCAGGGCTCCA
CCGCGCGCCCGGTACGGGGCCCTTCCTTAGCTCACCGGGGACCCAAGGCCCATGGG
CCACACCCAGGGTGGCCCTGACGTCCCCGTAACCCATGCGGATACAGTCTCGTGGGT
GGGCGAGGGTTCCCTGAGTGGGTGGGCTTCCGGGGTGGACAGAGTCCCATGGGTGG
GGGGGGTCACATGGGTAGGCGGGGTCCCGGGGGTGGGGTCCCGTGGGTGGGTGTGG
TCCCCGATGGGCGGGGGTCCTGTGGGCGTGGTCACAGGGTGGGCGTGGTCCCGCGG
TGGGCGGAGTCCCGTGGGTGGGCGGGGTCCCGGGGTGGGTGCAAGGAGAGCGGTCC
CAGGATGAGTGGGGATCCCGGGGTAGGCGGGGCCCTGGGTGGGCGGGGCTGCGCAC

The haplotype shown in Figure 5 is based on previously demonstrated polymorphic sites that were included in commercial DNA marker testing arrays, panels, or chips. The MI SNP 79613592 was not described and included in commercial genotype panels prior to discovery of the association with MI.

### MI Haplotype Determination

Disclosed herein are methods and composition for identifying a bovine, including a young or adult bovine animal, an embryo, a semen sample, an egg, a fertilized egg, a zygote, fetus, or other cell or tissue sample therefrom, to determine whether said bovine possesses a motor impairment (MI) haplotype. Determination of the MI haplotype, located at position 78732954 to 80748266 bp on chromosome 16 of Holstein cattle, provides a quick, accurate, and effective means to identify whether an animal should be used for breeding stock, whether an animal is likely to develop motor impairment, and whether further progeny testing should be performed.

DNA markers have several advantages over phenotype analysis; segregation is easy to measure and is unambiguous, and DNA markers are co-dominant, i.e., heterozygous and homozygous animals can be distinctively identified. Once a marker system is established selection decisions can be made very easily, since DNA markers can be assayed any time after a DNA-containing sample, such as a blood or tissue sample, can be collected from the individual infant animal, or even earlier by testing embryos in vitro if very early embryos are collected. The use of marker assisted genetic selection will greatly facilitate and accelerate cattle breeding programs.

Also disclosed is a breeding method whereby haplotype testing as described below is conducted on bovine embryos, young adults, or adult cattle, and based on the results, certain animals are either selected for or removed from the breeding program. Preferably, individuals negative for the MI haplotype are selected. Preferably, these individuals are homozygous with regard to the negative MI haplotype. For example, the individual is homozygous with regard to a negative MI haplotype.

The present disclosure relates to analyzing a nucleic acid sample from a bovine, including a young or adult bovine animal, an embryo, a semen sample, an egg, a fertilized egg, or a zygote, or other DNA-containing biological sample (e.g., blood, cell or tissue sample therefrom), to determine whether said bovine possesses one of the haplotypes disclosed herein, indicative of motor impairment. The nucleic acid sample is representative of DNA at position 78732954 to 80748266 bp on chromosome 16 (the "MI region"), or a fragment thereof. The nucleic acid can comprise genomic DNA, cDNA, or RNA.

In some embodiments, the method comprises haplotyping nucleic acid at bovine DNA at position 78732954 to 80748266 bp on chromosome 16 (the "MI region") or fragment thereof, for at least one copy of the MI haplotype and optionally assigning to the individual a bovine MI haplotype score. The method may be used to identify the haplotype of both copies of the MI region in the animal, and assigning a haplotype pair to the animal.

In some embodiments, a haplotyping method comprises examining one copy of the MI region, or a fragment thereof, to identify the nucleotide at two or more polymorphic sites in that copy to assign a haplotype to the individual.

In some embodiments, a haplotyping method comprises examining one copy of the MI region, or a fragment thereof, to identify a single polymorphic site in that copy. In some embodiments, the single polymorphic site comprises the MI SNP, and is bp 79613592 of bovine chromosome 16 and is shown in SEQ ID NO: 1/2, where underline represents an intron:

GGGGCCCTCTGGCCCCTCACCTGCATGC[C/T]GATGACCGCGTAGATGAAGAAGAGC
ATGACG (SEQ ID NO:1 and 2).

As will be readily appreciated by those skilled in the art, if an MI region is cloned and sequenced any individual clone will typically only provide haplotype information for a portion of one of the two MI region copies present in an individual. If haplotype information is desired for both copies of the individual's entire MI region, additional MI clones will need to be examined.

Further provided is a method for genotyping the bovine MI region, comprising determining for the two copies of the MI region present the identity of the nucleotide pair at one or more polymorphic sites, wherein the one or more polymorphic sites (PS) have the alternative alleles associated with the MI phenotype.

In some embodiments, a genotyping method comprises examining both copies of the MI region, or a fragment thereof, to identify the nucleotide pair at one or more polymorphic associated with the MI phenotype in the two copies to assign a genotype to the individual. In some embodiments, "examining a region" may include examining one or more of: DNA containing the region, mRNA transcripts thereof, or cDNA copies thereof. As will be readily understood by the skilled artisan, the two "copies" of a gene, mRNA or cDNA, or fragment thereof in an individual may be the same allele or may be different alleles. In another embodiment, a genotyping method of the invention comprises determining the identity of the nucleotide pair at each of the polymorphic sites associated with the MI phenotype.

In some embodiments, genotyping comprises an evaluation of one or more of the alleles in the shaded region of FIG. 5 (78732954 to 80748266 bp of bovine chromosome 16). In some embodiments, genotyping comprises evaluating the nucleic acid sample for the MI SNP at bp 79613592 of bovine chromosome 16 as shown below (underlined sequence represents intronic sequence):

GGGGCCCTCTGGCCCCTCACCTGCATGC[C/T]GATGACCGCGTAGATGAAGAAGAGC
ATGACG (SEQ ID NO: 1 and 2).

The present disclosure further provides systems, platforms, and kits for genotyping and/or haplotyping a bovine sample, the system or kit comprising in a container one or more nucleic acid molecules designed for detecting the one or more polymorphisms associated with the MI phenotype, and optionally at least another component for carrying out such detection. In some embodiments, a kit comprises at least two oligonucleotides packaged in the same or separate containers. The kit may also contain other components such as hybridization buffer (where the oligonucleotides are to be used as a probe) packaged in a separate container. Additionally, or alternatively, where the oligonucleotides are to be used to amplify a target region, the kit may contain, preferably packaged in separate containers, a polymerase and a reaction buffer optimized for primer extension mediated by the polymerase, such as PCR.

In some embodiments, a system, platform, or kit may be configured to detect a mutant protein, such as a mutant calcium voltage-gated channel subunit alpha1 S gene (CACNA1S). In some embodiments, the mutant alters a GGC codon to AGC which facilitates a glycine to serine amino acid substitution.

Thus, in some embodiments, a system, platform or kit comprises immunoassay reagents for detecting a mutant protein, including one or more antibodies and buffers, wherein the primary or secondary antibody may have an attached enzyme that produces a color when a substrate for the enzyme is added and reacts with the enzyme. The enzyme that produces color may be linked to either the antigen or a primary or secondary antibody.

Also disclosed is a breeding method is provided whereby haplotyping and/or genotyping as described above is conducted on bovine embryos, and based on the results, certain cattle are either selected or dropped out of the breeding program. In some embodiments, individuals carrying an MI negative haplotype are selected for continued and future breeding.

Through use of the linked marker loci, the different haplotypes can be manipulated in genetic improvement programs by procedures termed "marker assisted selection" (MAS), for genetic improvement within a breeding nucleus; or "marker assisted introgression" for transferring useful alleles from a resource population to a breeding nucleus (Soller 1990; Soller 1994).

### Genotyping Assays

### I. Hybridization-based Methods

### A. Arrays

In some embodiments, a nucleic acid sequence(s) comprising the MI SNP can be incorporated into a genotyping platform, comprising a panel or nucleic acid target array. As used herein, "panel" and "array" are used interchangeably and refer to a solid-state substrate, typically a glass, plastic or silicon slide, used to assay and detect single nucleotide polymorphisms from DNA samples in parallel, and genotypic data is output. Numerous panel/array platforms are commercially available and are well known in the art. By way of example, array genotyping platforms directed to cattle include, but are not limited to Neogen's BOVUHDV03 and GeneSeek Genomic Profiler (GGP) Bovine 150K; Illumina's bovine array beadchips, such as BovineSNP50 DNA Analysis BeadChip and BovineHD DNA Analysis Kit; and ThermoFisher's Axiom Bovine Genotyping 100K Array and Axiom Genome-Wide BOS 1 Bovine Array, Affymetrix platforms, among others. In some embodiments, nucleic acid sequence(s) comprising the MI SNP can be incorporated into such commercially available genotyping platforms. Alternatively, nucleic acid sequence(s) comprising the MI SNP can be incorporated into a custom designed array designed to assay specific pools of SNPs, some of which SNPs may overlap with a commercially available platform. Incorporation of nucleic acid sequence(s) comprising the MI SNPs depends on the chemistry of the array platform, but follows the principles of the array technology. For example, nucleic acid sequence(s) comprising the MI SNPs are used to design one or more complementary probe sequences, wherein the probe is affixed to the array.

By way of example, these array platforms represent a subset of a larger number of commercially available array platforms varying in SNP content, density (number), chemistry, throughput (number of samples that can be assayed simultaneously), or other properties. However, arrays generally rely on the hybridization of fragmented single-stranded sample DNA to short single-stranded probes, also known as oligonucleotides, which are typically 25-50 bp long. The probes are typically attached, printed, or synthesized directly onto the array surface in a grid-like fashion onto a two-dimensional solid-state surface, such as glass, plastic or silicon. The probe sequences are designed using apriori DNA sequence knowledge, which may have been collected from a panel of individuals representing genotypic diversity for that species, to artificially synthesize sequences that are complementary to the sample DNA fragment sequences. Hybridization depends on the binding of nucleotide bases with their complementary bases, where the probe and sample DNA are complementary in sequence, and thus hybridize. Even strands of DNA having less than a perfect match can hybridize. Typically, the DNA sample sequence matches the probe at 100%, in some embodiments, 99%, 98%, 97%, 96%, 95%, etc. down to 80%.

Array platforms typically utilize fluorescent dye to generate signals that are created when the dye is excited by a laser and the emission spectra detected by a camera, whereby the signals report specific nucleotide identities at the assayed basepair position within the probe sequence. The resulting raw images are normalized to subtract background signals and converted into genotypic data with confidence scores using computational algorithms (LaFramboise, Thomas, 2009). Variations of array technology exist, and the details provided herein are included by means of example but not by way of limitation.

### B. Molecular Beacons

In some embodiments, a nucleic acid sequences(s)comprising the MI SNP can be genotyped using probe oligonucleotides configured as molecular beacons. Molecular beacons are single-stranded oligonucleotide probes that are designed with complementary sequences on each end of the probe sequence, which creates a stem-loop structure. A fluorophore is added to one end of the probe and a fluorescence quencher to the other end. When in close proximity, such as is achieved when the molecule remains in the stem-loop state, no fluorescence is emitted. However, when the probe sequence hybridizes to its target sequence (the complementary sample DNA sequence), the stem-loop denatures, thereby separating the fluorophore and quencher, and allowing fluorescence. An individual's genotype at a particular position can be determined by designing one molecular beacon for one allele and another beacon for an alternate allele, where the probe wavelengths for each beacon differ. Different alleles can be distinguished by the different wavelengths each fluorophore emits.

### C. Dynamic allele-specific hybridization (DASH)

In some embodiments, a nucleic acid sequence(s) comprising the MI SNP can be genotyped using DASH genotyping, which is based on detecting differences in melting temperatures of double-stranded DNA when there is a mismatched nucleotide. A fluorescent marker only emits a signal when the molecule is bound to dsDNA, which is formed between an allele-specific oligonucleotide and the sample DNA. As the temperature of the reaction is increased, the dsDNA denatures into ssDNA, and the marker no longer fluoresces.

### II. Enzyme Based Assays

A number of enzymes can be used to develop SNP genotyping assays, including DNA polymerase, DNA ligase and nucleases.

### A. Polymerase Chain Reaction (PCR) based methods

In some embodiments, a genotyping assay includes an amplification step, e.g., to amplify nucleic acid from a subject sample, wherein the nucleic acid comprises the MI SNP. The skilled person understands how to design primers and a polymerase chain reaction (PCR) protocol to amplify the nucleic acid target of interest, such as a sequence comprising the MI SNP. The primers may be designed to be complementary to the sequences flanking the MI SNP, such that the MI SNP and any sequence 3' of the forward primer and 5' of the reverse primer is amplified via PCR. The primer sequences may be variable in length, sequence composition or position relative to the MI SNP, and may amplify variable length amplicons of varying sequence compositions, wherein the amplicon contains the MI SNP. PCR-based genotyping technologies include but are not limited to realtime or quantitative PCR (qPCR) and end-point genotyping.

In some embodiments, a nucleic acid sequence(s) comprising the MI SNP can be genotyped using real-time qPCR. Real-time qPCR approaches are well known in the art, and generally rely on measuring PCR product accumulation over each PCR cycle, i.e., in real time, as opposed to after a specified number of PCR cycles as is done with PCR. qPCR typically relies on a reporter molecule/enzyme to determine the amount of PCR product present relative to a control in real time. SYBR green is one type of non-specific dye that can be used to report on PCR product accumulation by binding to dsDNA and fluorescing when bound. Hybridization probes may also be employed, wherein the probes consist of oligonucleotides complementary to sequences internal to the PCR product. The probes are tagged with different fluorescent dyes, one dye at the 3' end of one probe and a different dye at the 5' end of a second probe. As PCR products accumulate, the probes bind to the target DNA, which brings them close enough together to catalyze a fluorescence resonance energy transfer (FRET), wherein energy is transferred from the shorter wavelength dye (the donor) to the longer wavelength dye (the acceptor). The PCR product amount can be estimated based on the wavelength and intensity of the fluorescence detected.

In another embodiment, a nucleic acid sequence(s) comprising the MI SNP can be genotyped using end-point genotyping, which involves measuring a PCR amplification product after a specified number of PCR cycles. One example of an end-point PCR genotyping assay is LGC Biosearch's Kompetitive allele specific PCR, or KASP, which is based on allele-specific PCR and fluorescence resonant energy transfer (FRET). Two allele-specific forward primers, each labeled with a distinct fluorescent dye, and a common reverse primer allow for amplification of the sample DNA regardless of which allele it contains. The SNP genotype is determined by the emission spectra detected, where a homozygous individual is indicated by a signal consisting of one of two possible spectra, depending on the allele, and a heterozygous individual is indicated by a signal containing both possible spectra.

### B. Restriction Fragment Length Polymorphisms (RFLPs)

In some embodiments, nucleic acid sequences comprising the MI SNP can be genotyped using restriction fragment length polymorphisms (RFLPs). This assay comprises digesting a DNA sample into fragments by one or more restriction enzymes, where the resulting fragments can be separated by gel electrophoresis. The patterns of banding determine the composition of nuclease cleavage sites found within the DNA sample sequence, which ultimately reports on the original DNA sequence.

### C. Flap endonuclease (FEN) (Invader Assay)

In some embodiments, a nucleic acid sequence(s) comprising the MI SNP can be genotyped using flap endonuclease (FEN). FEN is an endonuclease that recognizes and cleaves a specific molecular structure. One example of an assay that that utilizes a FEN called cleavase is the Invader assay. The Invader assay is well known in the art, and basically involves designing an oligonucleotide Invader probe that is complementary to the 3' end of the variable SNP site. The last nucleotide of the Invader probe is a non-matching base that overlaps the variable SNP site. A second allele-specific probe is designed to be complementary to the 5' end of the variable SNP site but extends past the 3' side of the site. If the sample DNA and the second allele-specific probe are complementary in sequence, they will hybridize, and in the presence of the invader probe form a three-dimensional complex that is recognized and cleaved by FEN. Fluorescence resonance energy transfer (FRET) technology can be used to detect the cleavage reaction by triggering a secondary cleavage reaction. The secondary cleavage reaction occurs between a quencher molecule that is attached to the 3' end of the second allele-specific probe, and a fluorophore that is attached to the 5' end of the same probe. The primary cleavage by FEN separates the quencher from the fluorophore and thus causes a detectable signal. The invader assay is only one such genotyping assay and is meant to serve as an example. Variations of the invader assay include but are not limited to Serial Invasive Signal Amplification Reaction (SISAR), which allows both SNP alleles to be tested in a single reaction.

### D. Primer Extension

In some embodiments, nucleic acid sequences comprising the MI SNP can be genotyped using primer extension or single base extension, a mini-sequencing technique. A detection primer is designed to target a sequence directly upstream of the SNP. The 3' end of the oligonucleotide is then extended by a single base using dideoxynucleotide triphosphates (ddNTPs), wherein each ddNPT base corresponds to a different fluorescent dye. The result is detection of up to four alleles, including the discrimination of homozygous and heterozygous genotypes. A number of detection platforms are able to detect the SNP, such as mass spectrometry (matrix-assisted laser desorption/ionization time of flight (MALDI-TOF), capillary electrophoresis, pyrosequencing, flow cytometry or fluorescence plate readers.

### E. 5'-nuclease (TaqMan)

In some embodiments, nucleic acid sequences comprising the MI SNP can be genotyped using a 5'-nuclease. TaqMan probes utilize a variation of fluorescence resonance energy transfer (FRET) technology where a fluorophore is covalently attached to the 5' end of an oligonucleotide probe, and a quencher is covalently attached to the 3' end of the same probe. When PCR products accumulate, the probe binds to the PCR product during a denaturation cycle, and as Taq polymerase synthesizes a new strand, it encounters the hybridized probe. The exonuclease activity of Taq releases the fluorescently tagged nucleotide at the 3' end of the probe. The fluor is now released into solution where it will fluoresce because it is no longer close enough to the quencher to carry out FRET.

### F. Oligonucleotide Ligation Assay

In some embodiments, nucleic acid sequences comprising the MI SNP can be genotyped using an oligonucleotide ligation assay. This assay depends on the enzyme DNA ligase to ligate two DNA probes at the SNP site, wherein ligation occurs if both probes have perfect base pair complementarity to the target sequence. The first probe sequence is an allele-specific probe designed to be complementary to the target DNA so that its 3' base corresponds to the target SNP, and the second probe sequence is designed to hybridize to the sequence directly adjacent to the SNP in the other direction so that its 5' end is provided for the ligation reaction. Ligation will only occur if the probes match and hybridize with the target DNA. Any platform that can detect a difference in sequence length can be used to determine if the product is ligated or un-ligated, including gel electrophoresis, MALDI-TOF mass spectrometry, capillary electrophoresis. Alternatively, the oligonucleotides can be tagged and/or labeled so as to index samples and allow for high-throughput sequencing of the ligated products, wherein the genotypes of said products can be determined.

### IV. Nucleic Acid Sequencing

In some embodiments, the MI SNP may be detected by sequencing a nucleic acid sequence(s) comprising the MI SNP. Sequencing methodologies are broad, and a number of variations of the platform and chemistry exist. For example, first generation sequencing technologies utilize Sanger sequencing and rely on the electrophoretic separation of chain-termination products. Later generation sequencing technologies, including Next Generation Sequencing (NGS), are well-known in the art, and generally describe massively parallel sequencing via clonally amplified DNA templates in a flow cell. Sequencing methods that can be employed include but are not limited to whole genome sequencing (WGS), targeted resequencing, RNASeq, Exome Capture/ Sequencing and genotype-by-sequencing (GBS), wherein the nucleic acid(s) sequenced can be DNA or RNA. These methods require use of a sequencing platform, where factors such as chemistry type, desired level of multiplexing, desired data output amount, and run-time should be considerations in choosing a platform. Examples of sequencing platforms include but are not limited to the Roche 454, Illlumina MiSeq, Illumina HiSeq, Life Technologies SOLiD4 and Pacific Biosciences SMRT platforms.

### V. Mass Spectrometry

Mass spectrometry methods determine the mass of an ionized molecule by measuring the molecule's migration rate in an electric field, where smaller molecules travel faster. Some types of spectrometers are able to separate different sized fragments and further fragmentize them to sequence the oligonucleotide.

### VI. Haplotype Assays

Detection of a haplotype can be achieved by detection of one or more polymorphisms comprising the haplotype, wherein the MI haplotype comprises 120 markers ranging from 78,732,954 to 80,748,266 bp, including the MI SNP, as shown in **Table** 2. MI haplotype marker genotypes can be determined using any of the genotyping methods above, or a combination thereof, e.g., one or more assays may be designed to detect the genotype(s) of all or a portion of the 120 markers comprising the MI haplotype. The MI SNP may be genotyped using the same assay as is used for detecting the genotype(s) of the MI haplotype marker(s), or it may be genotyped using a different assay. In one embodiment, all or a portion of the 120 markers comprising the haplotype, optionally including the MI SNP, and additional markers in linkage with the MI SNP may be added to a commercially available array platform, such as those described earlier.

### Protein Detection Assay

An alternative method for detecting a mutant individual is through use of an immunoassay, such as ELISA (enzyme-linked immunosorbent assay), which can detect the presence of a protein of interest or a mutant protein of interest. Antibodies designed to bind the protein in question are added to a liquid sample, wherein said binding or the binding of a secondary antibody causes a detectable signal that can then be measured. In this way, a mutant protein can be detected by antibodies specific to the mutant versus the wild-type protein. Both the wild-type and mutant versions of the protein encoded by the CACNA1S gene could be detected using this type of assay.

In some embodiments, a nucleic acid of the present disclosure, comprising the MI SNP, is added to an existing genotyping platform, panel or array. In some embodiments, the nucleic acid is added to the platform, panel, or array in double-stranded form. In some embodiments, the nucleic acid is added to the platform, panel, or array in single-stranded form. In some embodiments, the nucleic acid is single-stranded and comprises a sense strand of DNA including the MI SNP. In some embodiments, the nucleic acid is single-stranded and comprises the antisense strand of DNA including the MI SNP. In some embodiments, the nucleic acid is single-stranded and comprises an RNA including the MI SNP.

In some embodiments, the nucleic acid comprises SEQ ID NO: 1 and/or SEQ ID NO: 2, the complements thereof, or reverse complements thereof. In some embodiments, the nucleic acid comprises a fragment of SEQ ID NO 1 and/or SEQ ID NO: 2, the complements thereof, or reverse complements thereof, wherein the fragment comprises the MI SNP. The fragment includes at least 10 nucleotides 5' or at least 10 nucleotides 3' of the MI SNP position. In some embodiments the fragment includes at least 10 nucleotides 5' and at least 10 nucleotides 3' of the MI SNP position. In some embodiments, the nucleic acid comprising the MI SNP includes at least about 10, at least about 15, at least about 20, at least about 25, at least about 30, at least about 35, at least about 40, at least about 45, at least about 50, at least about 55 at least about 60, at least about 65 at least about 70, at least about 75, at least about 80, at least about 85, at least about 90, at least about 95, or at least about 100 nucleotides 5', 3', or both 5' and 3' of the MI SNP, or any combination of lengths for the 5' and 3' sides of the MI SNP. In some embodiments, the nucleic acid or fragment thereof comprising the MI SNP has about 70%, about 75%, about 80% about 85%, about 90% about 91%, 92%, 93%, 94%, 95%, 96%, 97%, 97%, 99% identity to SEQ ID NO: 1, SEQ ID NO: 2, a complement thereof, reverse complement thereof, or a fragment of any of these. In some embodiments, a nucleic acid comprising the MI SNP deviates from SEQ ID NO: 1 or SEQ ID NO: 2 by virtue of variation in the genetic code. That is, the variant nucleic acid sequence (or fragment) encodes the same amino acid sequence, although the nucleic acid sequence may differ from that of SEQ ID NO: 1, SEQ ID NO: 2, a complement thereof, or a reverse complement thereof.

In some embodiments, the nucleic acid comprising the MI SNP also includes a detectable marker. The detectable marker may embody a number of formats, such as a dye that binds double stranded DNA of the nucleic acid comprising the MI SNP, or fluorescently labeled probes that hybridize with the nucleic acid comprising the MI SNP.

The following examples are intended to illustrate preferred embodiments of the invention and should not be interpreted to limit the scope of the invention as defined in the claims.

### EXAMPLES

### Example 1. Identification of a haplotype associated motor impairment in Holstein calves.

### Background

Holstein genomic selection programs screen for several genetic recessives associated with stillbirth and late term abortions (Agerholm et al., 2001; Charlier et al., 2012) and with neonate survival (Shuster et al., 1992; Schütz et al., 2016; Basiel et al., 2020). Genomic testing has also accelerated discovery of genetic recessives that result in embryonic loss (Fritz et al., 2013, 2018; McClure et al., 2014; Adams et al., 2016) by screening for regions where a given haplotype is not observed in a homozygous state when expected to be present based on haplotype frequency (VanRaden et al., 2011).

While genomic testing has facilitated rapid genetic improvement in addition to identification of genetic recessives, there has also been an acceleration of inbreeding when viewed on an annual and on a generational basis (Makanjuola et al., 2020). The current rise follows a period of contraction in male lineages due to the widespread adoption of artificial insemination

(Dechow et al., 2020). Elevated inbreeding and small effective population sizes have increased the increased likelihood that new genetic recessive conditions will manifest in a significant number of animals (Fritz et al., 2013). Recently, motor impairment in newborn calves has been observed by veterinarians and farm staff in multiple states. Genetic defects are a prime contributor to neuromuscular defects in humans and other species (Laing, 2012), and could underlie the problem observed in calves. Therefore, the objectives of this study were to conduct a genome wide association study of affected calves and normal family members to determine if a genetic origin was likely and to establish plausibility of genetic inheritance based on shared ancestors.

### Materials and Methods

*Sample population.* Table 1 provides details on the number of affected calves and the genotyped control population. Affected calves (34 total) were recorded on farms from New York state (farms 1 and 2), Florida (farm 3), and Pennsylvania (farm 4). The condition was first noted in December of 2019 with the latest observations from November of 2021. The affected calves were all unable to stand without assistance at one more time during the neonatal period with varying severity, recovery, and relapse patterns. Some were unable to stand for the first 24 hours after birth, whereas others were able to stand initially and then lost the ability to do so within the first month of life. There were 21 calves euthanized whereas 7 calves were considered recovered and 6 were unthrifty with poor growth at the time of data collection. Table 1 shows the number of calves with motor impairment and recovery status by farm, number of impaired calves that resulted from embryo transfer, number of genotyped impaired calves, number of genotyped control siblings, and number of parent or other relatives with genotypes.

**Table 1: Sample Population**

| | | Farm 1 | Farm 2 | Farm 3 | Farm 4 |
|---|---|---|---|---|---|
| Motor impairment (n) | | 10 | 3 | 17 | 4 |
| | Recovered (n) | 1 | 0 | 5 | 1 |
| | Unhealthy (n) | 1 | 0 | 5 | 0 |
| | Died/euthanized (n) | 8 | 3 | 7 | 3 |
| Sire × dam combinations (n) | | 10 | 2 | 3 | 2 |
| Embryo transfer | | 0 | 3 | 16 | 3 |
| Impaired calf genotypes | | 1 | 1 | 12 | 4 |
| Control sibling genotypes | | 0 | 0 | 22 | 0 |
| Parent genotypes | | 0 | 0 | 3 | 0 |
| Other relative genotypes | | 0 | 0 | 0 | 1 |

Necropsy was performed on calves at the Cornell University College of Veterinary Medicine and the University of Florida College of Veterinary Medicine. Histological examination of muscle and peripheral nerves followed biopsy of the right bicep and right brachial plexus from one farm 3 calf. Blood samples were collected from four calves on farm 1 to determine if cholesterol deficiency, anemia, or selenium deficiency might contribute to the motor impairment, and multiple blood samples were collected on farm 3 to evaluate potential selenium deficiency.

Calves were from 17 sire × dam combinations and 22 calves resulted from in-vitro fertilization with full sibling families of 13 (farm 3), 3 (farm 3 and 4), and 2 (farm 2). All farm 3 calves were sired by a single bull mated to 3 dams.

*Genomic and pedigree analysis.* Tissue, hair or blood samples were available for genotyping from 18 affected calves ranging from 1 to 12 per farm with the remaining calves destroyed before sample collection could occur. For a control population, 22 healthy full siblings from two (17 and 5 calves, respectively) farm 3 dams were sampled. Additionally, the sire of the farm 3 calves and a full sister to the dam of three farm 4 calves were sampled. All samples were genotyped for 139,376 DNA markers (BOVUHDV03, Neogen, Lincoln, NE). The genotypes of two farm 3 dams were imputed with findhap.f90 (v3; Vanraden et al., 2011) and included in subsequent analyses.

Motor impairment was recorded as 1 for 26 unaffected animals including the control calves and genotyped relatives, or 2 for the 18 affected calves. Association analysis was conducted in PLINK (v1.9; Chang et al., 2015; www.cog-genomics.org/plink/1.9/). A marker call rate of ≥99% was imposed, leaving 101,907 makers for analysis. The initial association analysis was comprised of an allelic chi-square test (frequency of allele A versus frequency of allele B) assuming 1 degree of freedom with p-values generated using Fisher's exact test. This was followed with a dominance model (genotype AA and AB versus BB) with a 1 degree of freedom chi-square test. Multiple comparison testing was considered by implementing a false discovery-rate (FDR) p-value to declare genome-wide significance at FDR P≤0.05. Homozygosity screening was also performed to identify runs of homozygosity.

Thirty-three of the affected calves had a known sire and were included in a pedigree analysis. Two generations of sire identification (sire and maternal grand-sire) were available for nine calves from farm 1 whereas a minimum of six generations (calf's sire plus sires for 5 generations of dams) was available for all remaining calves. All known ancestors were traced separately for the sire and dam of each affected calf and the number of calves where an ancestor was present in the sire and dam lineage was determined to identify plausible common ancestors. Imputed genotypes of potential ancestors were derived using findhapf90 using the affected calve and control genotypes plus a population of 7622 Holsteins with genotype densities ranging from 3K to 150K.

### Results

*Clinical findings.* Necropsy failed to determine a cause for motor impairment for any of the calves; there was no evidence of cholesterol deficiency, selenium deficiency, or anemia based on serum testing results. A definitive disease diagnosis was not supported by the histological examination of muscle and peripheral nerves; however, the intramuscular nerve branches of the bicep biopsy showed moderate subperineural edema with scattered macrophage infiltration of the endoneurium, endomysium and perimysium. Similarly, moderately severe edema was noted in the endoneurium and subperineurium along with scattered mononuclear cell infiltration of nerve fascicles of the brachial plexus. The behavior and appetite of the calves was normal and there were no obvious clinical symptoms preventing proper motor function.

*Genome wide association.* A Manhattan plot demonstrating the significance level for each marker according to the dominance model is shown in Figure 1 with a peak apparent at the end of chromosome 16. In total, 78 markers in the region were significant (FDR P≤0.05) over a 5.1 million bp region that ranged from 75,332,437 to 80,448,457 bp (ARS-UCD 1.2). The allelic model returned similar results with more significant markers (88) over a slightly expanded range (5.7 million bp). The affected calves had a shared run of homozygosity that spanned 2.1 million bp from 78,732,954 to 80,748,266; there was one control calf homozygous over the same region. Figure 5 contains a list of DNA markers for the region along with genotype frequencies for affected calves. The highlighted sequences represent allele positions in MI affected animals.

The association of a 120-marker haplotype ranging from 78,732,954 to 80,748,266 bp with the motor impairment phenotype is shown in Table 2. All affected calves were homozygous and one of 26 controls was homozygous and was located on farm 3; 9 controls were homozygous for the alternate haplotype and 16 were heterozygous. The sire of the farm 3 calves and both dams were heterozygous for markers in the region.

**Table 2. Haplotype status for calves with (MI) and without (normal) motor impairment**

| | MI | Normal |
|---|---|---|
| MIH/MIH | 18 | 1 |
| MIH/- | 0 | 16 |
| -/- | 0 | 9 |

| | | |
|---|---|---|
| *MIH - putative motor impairment haplotype* | | |

A second region ranging from 9,585,914 to 10,754,653 bp that contained three significant markers was present on chromosome 20 with the dominance model (Figure 1) but the markers were not significant according to the allelic model. Seventeen of 18 affected calves were homozygous for the markers and the remaining affected calf was heterozygous. Of the controls, six were homozygous for the same allele, 18 were heterozygous, and 2 were homozygous for the alternate allele. Whether this indicates that the condition involves multiple loci or was a spurious result is not certain. In contrast to observations on chromosome 16, the affected calves were not all homozygous for the remaining markers spanning the region.

*Pedigree analysis.* The 33 affected calves with sire identification shared 63 known ancestors in their sire lineage born since 1990 with the most recent born in 2008 and 2010. For the maternal lineage, all 24 affected calves with at least 5 generations of known maternal sires traced to the bull born in 2008, versus 7 for the bull born in 2010. Additionally, the imputed genotypes suggested the 2008 sire was a carrier of the candidate region but not the 2010 sire. Therefore, the bull ROYLANE SOCRA ROBUST-ET (Robust) was considered the most likely ancestor of origin for these pedigrees. When expanded to include the remaining 9 calves with known maternal grand-sire, 30 of the calves could be traced to Robust. The pedigree with the most pathways from Robust to affected offspring is shown in Figure 2, and pedigrees for all genotyped calves or calves with at least 5 generations of sire known are reported in Figure 3. On the paternal side of the pedigrees, 30 of 33 affected calves could be traced to SEAGULL-BAY SUPERSIRE-ET (Supersire) whereas 23 of 24 of the maternal pedigrees could be traced to Supersire. Genotyping indicated that Supersire was also a carrier of the suspect haplotype.

### Discussion

Calves homozygous for a haplotype on chromosome 16 had elevated risk of motor impairment characterized by an inability to stand. While some calves partly recovered, the condition of most deteriorated and were euthanized, often from development of secondary conditions including pneumonia. Curiously, the calves appeared healthy other than impaired motor function.

Despite strong evidence for the candidate region, there is a degree of uncertainty due to the inconsistent development of the motor impairment phenotype, existence of a homozygous calf that was able to stand without assistance, and an additional region with significant markers; this raises the possibility that the identified genomic region is spurious and that the condition is non-genetic. Evidence for the region harboring a recessive allele was supported by high statistical significance, the presence of heterozygous haplotypes in genotyped parents, a shared homozygous haplotype across multiple affected families, and a plausible path of inheritance.

Observations in humans and animal models support a genetic basis for the motor impairment phenotype. Humans with defects in the CACNA1S gene are known to have muscle weakness (Fialho et al., 2018). In some instances, individuals are affected with Hypokalemic periodic paralysis which is associated with muscle paralysis that can last for a few minutes to a few days and can be triggered by diet and exercise. Similar to observations in calves with MI, CACNA1S mutations in humans result in variation in the frequency and duration of muscle weakness, as well as the age when the condition first appears. Genetic conditions in genes other than CACNA1S can also cause MI. Most neuromuscular disorders have a genetic origin and mutations in a wide variety of genes can cause disease (Laing, 2012). Genetic recessive conditions and dominant de-novo mutations have been associated with genetic pediatric neuromuscular disease in humans (Herman et al., 2021). A heritable juvenile-onset motor polyneuropathy was recently described in cats that was characterized by mononuclear cell infiltration of intramuscular nerve branches (Crawford et al., 2020). Mononuclear cell infiltration in skeletal muscle is a consistent characteristic of inflammatory myopathies (Ascherman, 2012).

A majority of the calves in this study resulted from IVF and embryo transfer. Epigenetic aberrations and developmental abnormalities including large offspring syndrome have been associated with IVF in multiple species (Ventura-Juncá et al., 2015). However, 11 calves were affected that resulted from AI and one resulted from natural mating. That observation coupled with strong a genomic association suggests that IVF is not the determining factor for development of motor inability. Nevertheless, a genotype × environment interaction with IVF increasing the likelihood that the phenotype will be expressed when the genetic defect is inherited cannot be discounted.

The rate of inbreeding on both and annual and generational basis increased considerably following the introduction of genetic selection (Dechow et al., 2020; Makanjuola et al., 2020), which has implications for long term selection response and increases the potential for genetic recessives to emerge (Fritz et al., 2013). The pedigree in Figures 2 and 3 demonstrate how a genetic recessive could rapidly proliferate given current rates of inbreeding. All the male ancestors in the pedigree are high ranking AI sires and demonstrate how a de novo mutation or low frequency mutation can be amplified rapidly. Supersire was listed as the most highly related sire of sons to the Holstein breed by the Council on Dairy Cattle Breeding (CDCB, 2021) following December of 2021 national genetic evaluations. If randomly mated to the current Holstein population, offspring would have an expected inbreeding of 11.9% based on pedigree and 11.5% based on genotypes. Pedigree and genomic future inbreeding values are 10.2% and 9.5%, respectively, for Robust.

Genomic testing has accelerated discovery of genetic recessives that result in embryonic mortality (VanRaden et al., 2011). Unfortunately, the haplotype screening method is not likely to identify conditions such as motor impairment because calves may appear normal at birth and be genotyped as part of a normal management routine. Genomic selection for heifer livability was introduced in national genetic selection programs in (Neupane et al., 2021); however, the heritability is low (<1%) resulting in modest levels of accuracy for genomic predictions. Moreover, heifers fail to survive for a variety of reasons such as death, injury, and reproductive failure. It is not clear that genomic evaluation of a quantitative trait with a large range of underlying conditions would help detect genetic recessives like that observed here unless a large proportion of calves were affected with a specific condition.

Current rates of inbreeding in the Holstein breed are favorable for rapid amplification of genetic defects. Support for a genetic defect on chromosome 16 that confers motor impairment includes genome wide significance that was shared across multiple mating combinations, heterozygous parent haplotypes, a plausible common ancestor for the affected pedigrees, and similar genetic conditions in other species. Despite strong evidence for the existence of a genetic condition, some caution is yet warranted because of the inconsistent phenotypic presentation of the calves. Future efforts to identify the exact location and type of mutation are needed to confirm to condition and facilitate genetic testing and selection.

### Example 2 - Sequence alignment and candidate mutation discovery

One affected calf that was homozygous for the recumbency haplotype, the sire of the affected calf, and a male ancestor that was phenotypically normal but known to be homozygous for the recumbency haplotype were sequenced at a depth of 30x using an Illumina (San Diego, CA) HiSeq pair-ended platform. The homozygous ancestor was identified at the USDA Animal Genomic Improvement Laboratory by tracing the haplotype of genotyped Holstein sires.

The sequence was aligned (Bowtie 2; https://bowtie-bio.sourceforge.net/bowtie2/index.shtml) to bovine chromosome 16 (ARS-UCD1; https://www.ncbi.nlm.nih.gov/assembly/GCF 002263795.2). Variants from the reference genome were considered candidate mutations if they were 1) homozygous in the affected calf, 2) heterozygous in the sire, and 3) not homozygous in the male ancestor. A SNP at bp 79613592 (C to T on forward strand, or G to A on the reverse strand) was identified that met the genotype criteria. The mutation and surrounding variants were heterozygous in the sire. The mutation was heterozygous in the male ancestor who was otherwise homozygous over the recumbency haplotype region. See FIG. 6.

The sequence of the positive DNA strand from bp 79613564 to 79613623 with the SNP indicated as [C] or [T] is:

GGGGCCCTCTGGCCCCTCACCTGCATG[C/T]TGATGACCGCGTAGATGAAGAAGAGC
ATGACG (SEQ ID NO: 1/2).

The mutation resides in the calcium voltage-gated channel subunit alpha1 S gene (CACNA1S) that is encoded on the negative strand. The missense mutation alters a GGC codon to AGC which facilitates a glycine to serine amino acid substitution. Ensembl (https://useast.ensembl.org/index.html) predictions (Table 3) indicate 9 potential transcripts which span 39 to 44 exons; the mutation resides in the 30^{th} to 32^{nd} exon of the transcripts.

**Table 3.**

| Table 3. Ensembl transcript predictions for gene ENSBTAG00000047491 | | | | |
|---|---|---|---|---|
| Ensembl transcript ID | Exon of mutation | Total exons | cDNA position | Protein position |
| ENSBTAT00000065901.2 | 31 | 43 | 3853 | 1285 |
| ENSBTAT00000069318.1 | 31 | 43 | 3853 | 1285 |
| ENSBTAT00000078499.1 | 31 | 43 | 3853 | 1285 |
| ENSBTAT00000065568.2 | 31 | 44 | 3853 | 1285 |
| ENSBTAT00000073862.1 | 31 | 41 | 3928 | 1310 |
| ENSBTAT00000070520.1 | 30 | 39 | 3970 | 1324 |
| ENSBTAT00000068152.1 | 32 | 42 | 4045 | 1349 |
| ENSBTAT00000086344.1 | 31 | 41 | 4474 | 1492 |
| ENSBTAT00000076942.1 | 31 | 41 | 4510 | 1504 |

The amino acid sequence corresponding to the mutated exon is shown in Table 4 for cattle and 5 other species. The highlighted amino acid indicates that the amino acid sequence is conserved across many species.

**Table 4.**

| Table 4. Species and amino acid sequence corresponding to the exon with the mutated coding sequence. | |
|---|---|
| Species | Amino acid sequence |
| Reference cattle sequence | ALPYVALLIVMLFFIYAVIGMQ (SEQ ID NO: 9) |
| Affected calf sequence | ALPYVALLIVMLFFIYAVISMQ (SEQ ID NO: 10) |
| Human | ALPYVALLIVMLFFIYAVIGMQ (SEQ ID NO: 9) |
| Goat | ALPYVALLIVMLFFIYAVIGMQ (SEQ ID NO: 9) |
| Horse | ALPYVALLIVMLFFIYAVIGMQ (SEQ ID NO: 9) |
| Dog | ALPYVALLIVMLFFIYAVIGMQ (SEQ ID NO: 9) |
| Mouse | ALPYVALLIVMLFFIYAVIGMQ (SEQ ID NO: 9) |

### CACNA1S mutations

CACNA1S enables calcium channel voltage gate activity and is highly expressed in skeletal muscle (https://www.ncbi.nlm.nih.gov/gene/682930). Mutations are known to cause periodic paralysis in humans (https://www.ncbi.nlm.nih.gov/gene/779) and mice (https://www.ncbi.nlm.nih.gov/gene/12292), which corresponds to the calf recumbency phenotype observed in Holstein calves.

The mutation was submitted to the Ensembl Variant Effect Predictor (https://useast.ensembl.org/Homo_sapiens/Tools/VEP). The SIFT (Sorting Intolerant From Tolerant) score confirmed that the mutation was deleterious (SIFT score range of 0 to 0.01) with a moderate projected impact, which corresponds to the partially penetrant nature of calf recumbency.

### Example 3 - exemplary assays and results

Genotypes of 16 animals for the C to T SNP at bp 79613592 in the CACNA1S gene (Table 5) were ascertained using a TaqMan assay. The genotyped animals included 6 recumbent calves that were homozygous for the MI haplotype (haplotype genotype=MI/MI); 4 normal animals that were previously determined to be carriers of the MI haplotype (haplotype genotype=+/MI); 4 recumbent calves that had not been genomically tested (haplotype genotype unknown), and two normal animals that were not genotyped and assumed to be free of the MI allele based on pedigree (haplotype genotype unknown). Two of the heterozygous animals were bulls that were ancestors to the recumbent calves and the remaining two were siblings of recumbent calves. All recumbent calves were homozygous for the MI allele regardless of previous genotype status. All animals heterozygous for the MI haplotype were heterozygous for the MI SNP. This demonstrated that directly testing for the SNP at bp 79613592 was able to ascertain the MI genotype correctly. The test was able to identify all expected genotypes (MI/MI, +/MI, and +/+) and a negative control demonstrated no detectable amplification.

**Table 5.**

| Phenotype | Number | Haplotype genotype | SNP genotype |
|---|---|---|---|
| Recumbent | 6 | MI/MI | MI/MI |
| Normal | 4 | +/MI | +/MI |
| Recumbent | 4 | Unknown | MI/MI |
| Normal | 2 | Unknown | +/+ |
| Negative control | 1 | No detection | No detection |

### References

Adams, H.A., T.S. Sonstegard, P.M. VanRaden, D.J. Null, C.P. Van Tassell, D.M. Larkin, and H.A. Lewin. 2016. Identification of a nonsense mutation in APAF1 that is likely causal for a decrease in reproductive efficiency in Holstein dairy cattle. J. Dairy Sci. 99. doi:10.3168/jds.2015-10517.
Agerholm, J.S., C. Bendixen, O. Andersen, and J. Arnbjerg. 2001. Complex vertebral malformation in Holstein calves. J. Vet. Diagnostic Investig. 13. doi:10.1177/104063870101300401.
Ascherman, D.P. 2012. Animal models of inflammatory myopathy. Curr. Rheumatol. Rep. 14. doi:10.1007/s11926-012-0245-7.
Basiel, B.L., A.L. Macrina, and C.D. Dechow. 2020. Cholesterol deficiency carriers have lowered serum cholesterol and perform well at an elite cattle show. JDS Commun.. doi: 10.3168/jdsc.2020-18587.
CDCB (Council on Dairy Cattle Breeding). 2021. Sires Highly Related To Holstein Cows in December, 2021. https://queries.usedcb.com/eval/summary/inbrd.cfm?R Menu= HO.k#StartBody. Accessed December 30, 2021.
Chang, C.C., C.C. Chow, L.C.A.M. Tellier, S. Vattikuti, S.M. Purcell, and J.J. Lee. 2015. Second-generation PLINK: Rising to the challenge of larger and richer datasets. Gigascience 4. doi:10.1186/s13742-015-0047-8.
Charlier, C., J.S. Agerholm, W. Coppieters, P. Karlskov-Mortensen, W. Li, G. de Jong, C. Fasquelle, L. Karim, S. Cirera, N. Cambisano, N. Ahariz, E. Mullaart, M. Georges, and M. Fredholm. 2012. A Deletion in the Bovine FANCI Gene Compromises Fertility by Causing Fetal Death and Brachyspina. PLoS One 7. doi:10.1371/journal.pone.0043085.
Crawford, K.C., D.L. Dreger, G.D. Shelton, K.J. Ekenstedt, and M.J. Lewis. 2020. Juvenile-onset motor polyneuropathy in Siberian cats. J. Vet. Intern. Med. 34. doi:10.1111/jvim.15963.
Dechow, C.D., W.S. Liu, L.W. Specht, and H. Blackburn. 2020. Reconstitution and modernization of lost Holstein male lineages using samples from a gene bank. J. Dairy Sci.. doi:10.3168/jds.2019-17753.
Fialho D, Griggs RC, Matthews E. 2018. Periodic paralysis. Handb Clin Neurol. 148:505-520. doi: 10.1016/B978-0-444-64076-5.00032-6. PMID: 29478596.
Fritz, S., A. Capitan, A. Djari, S.C. Rodriguez, A. Barbat, A. Baur, C. Grohs, B. Weiss, M. Boussaha, D. Esquerré, C. Klopp, D. Rocha, and D. Boichard. 2013. Detection of Haplotypes Associated with Prenatal Death in Dairy Cattle and Identification of Deleterious Mutations in GART, SHBG and SLC37A2. PLoS One 8. doi:10.1371/journal.pone.0065550.
Fritz, S., C. Hoze, E. Rebours, A. Barbat, M. Bizard, A. Chamberlain, C. Escouflaire, C. Vander Jagt, M. Boussaha, C. Grohs, A. Allais-Bonnet, M. Philippe, A. Vallée, Y. Amigues, B.J. Hayes, D. Boichard, and A. Capitan. 2018. An initiator codon mutation in SDE2 causes recessive embryonic lethality in Holstein cattle. J. Dairy Sci. 101. doi:10.3168/jds.2017-14119.
Herman, I., M.A. Lopez, D. Marafi, D. Pehlivan, D.G. Calame, F. Abid, and T.E. Lotze. 2021. Clinical exome sequencing in the diagnosis of pediatric neuromuscular disease. Muscle and Nerve 63. doi:10.1002/mus.27112.
LaFramboise, Thomas 2009. Single nucleotide polymorphism arrays: a decade of biological, computational and technological advances. Nucleic Acids Research 37(13). doi: 10.1093/nar/gkp552.
Laing, N.G. 2012. Genetics of neuromuscular disorders. Crit. Rev. Clin. Lab. Sci. 49. doi:10.3109/10408363.2012.658906.
Makanjuola, B.O., F. Miglior, E.A. Abdalla, C. Maltecca, F.S. Schenkel, and C.F. Baes. 2020. Effect of genomic selection on rate of inbreeding and coancestry and effective population size of Holstein and Jersey cattle populations. J. Dairy Sci. 103. doi:10.3168/jds.2019-18013.
McClure, M.C., D. Bickhart, D. Null, P. VanRaden, L. Xu, G. Wiggans, G. Liu, S. Schroeder, J. Glasscock, J. Armstrong, J.B. Cole, C.P. Van Tassell, and T.S. Sonstegard. 2014. Bovine exome sequence analysis and targeted SNP genotyping of recessive fertility defects BH1, HH2, and HH3 reveal a putative causative mutation in SMC2 for HH3. PLoS One 9. doi:10.1371/journal.pone.0092769.
Neupane, M., J.L. Hutchison, C.P. Van Tassell, and P.M. VanRaden. 2021. Genomic evaluation of dairy heifer livability. J. Dairy Sci. 104. doi:10.3168/jds.2020-19687.
Schütz, E., C. Wehrhahn, M. Wanjek, R. Bortfeld, W.E. Wemheuer, J. Beck, and B. Brenig. 2016. The Holstein Friesian lethal haplotype 5 (HH5) results from a complete deletion of TBF1M and cholesterol deficiency (CDH) from an ERV-(LTR) insertion into the coding region of APOB. PLoS One 11. doi:10.1371/journal.pone.0154602.
Shuster, D.E., M.E. Kehrli, M.R. Ackermann, and R.O. Gilbert. 1992. Identification and prevalence of a genetic defect that causes leukocyte adhesion deficiency in Holstein cattle. Proc. Natl. Acad. Sci. U. S. A. 89:9225-9229. doi:10.1073/pnas.89.19.9225.
VanRaden, P.M., J.R. O'Connell, G.R. Wiggans, and K.A. Weigel. 2011. Genomic evaluations with many more genotypes. Genet. Sel. Evol. 43. doi:10.1186/1297-9686-43-10.
VanRaden, P.M., K.M. Olson, D.J. Null, and J.L. Hutchison. 2011. Harmful recessive effects on fertility detected by absence of homozygous haplotypes. J. Dairy Sci. 94. doi:10.3168/jds.2011-4624.
Ventura-Juncá, P., I. Irarrázaval, A.J. Rolle, J.I. Gutiérrez, R.D. Moreno, and M.J. Santos. 2015. In vitro fertilization (IVF) in mammals: Epigenetic and developmental alterations. Scientific and bioethical implications for IVF in humans. Biol. Res. 48. doi:10.1186/s40659-015-0059-y.

Citations to a number of patent and non-patent references are made herein. In the event that there is an inconsistency between a definition of a term in the specification as compared to a definition of the term in a cited reference, the term should be interpreted based on the definition in the specification.

## Claims

1. A method comprising:
(a) generating genetic data from chromosomal DNA at position 78732954 to 80748266 bp on chromosome 16, or a fragment thereof, wherein the chromosomal DNA is present in a sample that has been obtained from a domestic bovine;
(b) identifying a motor impairment (MI) haplotype in the genetic data, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16; and
(c) selecting or removing the bovine as breeding stock based on the identification of (b).

2. The method of claim 1, wherein the bovine is:
(a) a heifer or cow;
(b) the bovine is a bull.

3. The method of any one of the preceding claims, wherein the bovine is phenotypically normal with respect to motor impairment.

4. The method of any one of the preceding claims, wherein the bovine is a Holstein, Holstein crossbred, or bovine of another breed with Holstein ancestry.

5. The method of any one of the preceding claims, wherein the sample:
(a) comprises semen or an oocyte
(b) comprises a tissue sample or a blood sample; or
(c) is derived from an embryo or fetus.

6. The method of any one of the preceding claims, wherein the genetic data comprises:
(a) the DNA sequence of at least one chromosome 16 of the bovine at position 78732954 to 80748266 bp, or a fragment thereof; or
(b) the DNA sequence of both chromosomes 16 of the bovine at position 78732954 to 80748266 bp, or a fragment thereof.

7. The method of any one of the preceding claims, wherein the MI haplotype comprises (ii) a T on the positive DNA strand at position 79613592 bp on chromosome 16.

8. The method of any one of the preceding claims, wherein generating genetic data comprises:
(a) sequencing the chromosomal DNA, or amplified copies thereof, optionally wherein sequencing comprises next generation sequencing; and/or
(b) an amplification step, such as a polymerase chain reaction.

9. A method comprising: detecting a homozygous negative for an MI haplotype in a genomic DNA sample from a bull, and identifying the bull as suitable for selective breeding to a female bovine, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

10. The method of claim 9, wherein:
(a) the female is also homozygous negative for the MI haplotype; or
(b) the bull is a Holstein bull, Holstein crossbred bull, or a bull from another breed with Holstein ancestry, and wherein the female bovine is a Holstein cow or heifer, Holstein crossbred cow or heifer, or a cow or heifer from another breed with Holstein ancestry.

11. An *in vitro* method, the method comprising: detecting a homozygous negative MI haplotype in a sample comprising genomic DNA sample obtained from a female bovine or an embryo derived from the female bovine, thereby identifying oocytes from the female as suitable for in-vitro embryo production, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

12. A method comprising: ascertaining the MI haplotype on a genomic DNA sample from a bull and a cow and identifying a homozygous negative female for the MI haplotype as suitable for mating to a
bull that is positive for the MI haplotype, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

13. A method comprising: ascertaining the MI haplotype from a genomic DNA sample obtained from a bull and identifying a homozygous negative bull for the MI haplotype and a female that is positive for the MI haplotype as suitable for corrective breeding, wherein the MI haplotype comprises:
(i) one or more of the alleles in the shaded portion of FIG. 5, with the shaded portion of FIG. 5 corresponding to 78732954 to 80748266 bp of bovine chromosome 16; and/or
(ii) a T on the positive DNA strand at position 79613592 bp on chromosome 16.

14. A composition, kit, or system comprising: at least one nucleic acid molecule, wherein the nucleic acid molecule comprises SEQ ID NO: 1, SEQ ID NO: 2, or a fragment of SEQ ID NO: 1 or SEQ ID NO: 2, wherein the fragment includes the MI SNP and wherein the fragment includes at least 10 nucleotides 5' or at least 10 nucleotides 3' of the MI SNP position.

15. The composition, kit, or system of claim 14, wherein:
(a) the nucleic acid molecule is attached to a solid support;
(b) the composition, kit or system further comprises an enzyme having polymerase activity, optionally wherein the enzyme comprises a Taq polymerase; or
(c) the nucleic acid molecule comprises a detectable marker.

## Patentansprüche

1. Verfahren, das umfasst:
(a) Erzeugen genetischer Daten aus chromosomaler DNA an der Position 78732954 bis 80748266 bp auf Chromosom 16 oder einem Fragment davon, wobei die chromosomale DNA in einer Probe vorhanden ist, die von einem Hausrind gewonnen wurde;
(b) Identifizieren eines Haplotyps für eine motorische Beeinträchtigung (Motor Impairment, MI) in den genetischen Daten, wobei der MI-Haplotyp umfasst:
(i) eines oder mehrere der Allele im schraffierten Bereich von Figur 5, wobei der schraffierte Bereich von Figur 5 den Basenpaaren 78732954 bis 80748266 auf dem Rinderchromosom 16 entspricht; und/oder
(ii) ein T auf dem positiven DNA-Strang an der Position 79613592 bp auf Chromosom 16; und
(c) Auswählen oder Aussortieren des Rindes als Zuchttier basierend auf der Identifizierung gemäß (b).

2. Verfahren nach Anspruch 1, wobei das Rind:
(a) eine Färse oder Kuh ist;
(b) ein Bulle ist.

3. Verfahren nach einem der vorhergehenden Ansprüche, wobei das Rind hinsichtlich einer motorischen Beeinträchtigung phänotypisch normal ist.

4. Verfahren nach einem der vorhergehenden Ansprüche, wobei das Rind ein Holstein, ein Holstein-Kreuzungstier oder ein Rind einer anderen Rasse mit Holstein-Vorfahren ist.

5. Verfahren nach einem der vorhergehenden Ansprüche, wobei die Probe:
(a) Sperma oder eine Eizelle umfasst;
(b) eine Gewebeprobe oder eine Blutprobe umfasst; oder
(c) von einem Embryo oder Fötus stammt.

6. Verfahren nach einem der vorhergehenden Ansprüche, wobei die genetischen Daten umfassen:
(a) die DNA-Sequenz mindestens eines Chromosoms 16 des Rindes an der Position 78732954 bis 80748266 bp oder ein Fragment davon; oder
(b) die DNA-Sequenz beider Chromosomen 16 des Rindes an der Position 78732954 bis 80748266 bp oder ein Fragment davon.

7. Verfahren nach einem der vorhergehenden Ansprüche, wobei der MI-Haplotyp (ii) ein T auf dem positiven DNA-Strang an der Position 79613592 bp auf Chromosom 16 umfasst.

8. Verfahren nach einem der vorhergehenden Ansprüche, wobei das Erzeugen genetischer Daten umfasst:
(a) Sequenzieren der chromosomalen DNA oder amplifizierter Kopien davon, optional wobei das Sequenzieren Next-Generation-Sequenzierung umfasst; und/oder
(b) einen Amplifikationsschritt, wie beispielsweise eine Polymerasekettenreaktion.

9. Verfahren, das umfasst: Nachweisen eines homozygot negativen MI-Haplotyps in einer genomischen DNA-Probe eines Bullen und Identifizieren des Bullen als geeignet für die selektive Zucht mit einem weiblichen Rind, wobei der MI-Haplotyp umfasst:
(i) eines oder mehrere der Allele im schraffierten Bereich von Figur 5, wobei der schraffierte Bereich von Figur 5 den Basenpaaren 78732954 bis 80748266 auf dem Rinderchromosom 16 entspricht; und/oder
(ii) ein T auf dem positiven DNA-Strang an der Position 79613592 bp auf Chromosom 16.

10. Verfahren nach Anspruch 9, wobei:
(a) das Weibchen ebenfalls homozygot negativ für den MI-Haplotyp ist; oder
(b) der Bulle ein Holstein-Bulle, ein Holstein-Kreuzungsbulle oder ein Bulle einer anderen Rasse mit Holstein-Vorfahren ist, und wobei das weibliche Rind eine Holstein-Kuh oder -Färse, eine Holstein-Kreuzungskuh oder -Färse oder eine Kuh oder Färse einer anderen Rasse mit Holstein-Vorfahren ist.

11. *In-vitro*-Verfahren, wobei das Verfahren umfasst: Nachweisen eines homozygot negativen MI-Haplotyps in einer Probe, die genomische DNA enthält, die von einem weiblichen Rind oder einem von diesem weiblichen Rind stammenden Embryo gewonnen wurde, wodurch Eizellen des weiblichen Rindes als geeignet für die *In-vitro-*Embryonenproduktion identifiziert werden, wobei der MI-Haplotyp umfasst:
(i) eines oder mehrere der Allele im schraffierten Bereich von Figur 5, wobei der schraffierte Bereich von Figur 5 den Basenpaaren 78732954 bis 80748266 auf dem Rinderchromosom 16 entspricht; und/oder
(ii) ein T auf dem positiven DNA-Strang an der Position 79613592 bp auf Chromosom 16.

12. Verfahren, das umfasst: Bestimmen des MI-Haplotyps an einer genomischen DNA-Probe eines Bullen und einer Kuh und Identifizieren eines homozygot negativen Weibchens für den MI-Haplotyp als geeignet für die Paarung mit einem Bullen, der positiv für den MI-Haplotyp ist, wobei der MI-Haplotyp umfasst:
(i) eines oder mehrere der Allele im schraffierten Bereich von Figur 5, wobei der schraffierte Bereich von Figur 5 den Basenpaaren 78732954 bis 80748266 auf dem Rinderchromosom 16 entspricht; und/oder
(ii) ein T auf dem positiven DNA-Strang an der Position 79613592 bp auf Chromosom 16.

13. Verfahren, das umfasst: Bestimmen des MI-Haplotyps aus einer genomischen DNA-Probe, die von einem Bullen gewonnen wurde, und Identifizieren eines für den MI-Haplotyp homozygot negativen Bullen und eines für den MI-Haplotyp positiven Weibchens als geeignet für die Korrekturzucht, wobei der MI-Haplotyp umfasst:
(i) eines oder mehrere der Allele im schraffierten Bereich von Figur 5, wobei der schraffierte Bereich von Figur 5 den Basenpaaren 78732954 bis 80748266 auf dem Rinderchromosom 16 entspricht; und/oder
(ii) ein T auf dem positiven DNA-Strang an der Position 79613592 bp auf Chromosom 16.

14. Zusammensetzung, Kit oder System, umfassend: mindestens ein Nukleinsäuremolekül, wobei das Nukleinsäuremolekül SEQ ID NO: 1, SEQ ID NO: 2 oder ein Fragment von SEQ ID NO: 1 oder SEQ ID NO: 2 umfasst, wobei das Fragment das MI-SNP enthält und wobei das Fragment mindestens 10 Nukleotide 5' oder mindestens 10 Nukleotide 3' der MI-SNP-Position umfasst.

15. Zusammensetzung, Kit oder System nach Anspruch 14, wobei:
(a) das Nukleinsäuremolekül an einen festen Träger gebunden ist;
(b) die Zusammensetzung, das Kit oder das System ferner ein Enzym mit Polymeraseaktivität umfasst, wobei das Enzym optional eine Taq-Polymerase umfasst; oder
(c) das Nukleinsäuremolekül einen nachweisbaren Marker umfasst.

## Revendications

1. Procédé comprenant :
(a) la génération de données génétiques à partir d'ADN chromosomique situé aux positions 78 732 954 à 80 748 266 pb sur le chromosome 16, ou un fragment de celui-ci, l'ADN chromosomique étant présent dans un échantillon prélevé sur un bovin domestique,
(b) l'identification d'un haplotype de déficience motrice (DM) dans les données génétiques, l'haplotype de DM comprenant :
(i) un ou plusieurs des allèles de la portion ombrée de la figure 5, la portion ombrée de la figure 5 correspondant aux positions 78 732 954 à 80 748 266 pb du chromosome 16 bovin, et/ou
(ii) un T sur le brin d'ADN positif à la position 79 613 592 pb sur le chromosome 16, et
(c) la sélection ou l'exclusion du bovin en tant qu'animal reproducteur en fonction de l'identification selon (b).

2. Procédé selon la revendication 1, dans lequel le bovin est :
(a) une génisse ou une vache,
(b) un taureau.

3. Procédé selon l'une quelconque des revendications précédentes, dans lequel le bovin présente un phénotype normal en ce qui concerne les déficiences motrices.

4. Procédé selon l'une quelconque des revendications précédentes, dans lequel le bovin est de race Holstein, un croisement de race Holstein ou un bovin d'une autre race ayant des ancêtres Holstein.

5. Procédé selon l'une quelconque des revendications précédentes, dans lequel l'échantillon :
(a) comprend du sperme ou un ovocyte,
(b) comprend un échantillon de tissu ou un échantillon de sang, ou
(c) provient d'un embryon ou d'un fœtus.

6. Procédé selon l'une quelconque des revendications précédentes, dans lequel les données génétiques comprennent :
(a) la séquence d'ADN d'au moins un chromosome 16 du bovin aux positions 78 732 954 à 80 748 266 pb, ou un fragment de celle-ci, ou
(b) la séquence d'ADN des deux chromosomes 16 du bovin aux positions 78 732 954 à 80 748 266 pb, ou un fragment de celle-ci.

7. Procédé selon l'une quelconque des revendications précédentes, dans lequel l'haplotype de DM comprend (ii) un T sur le brin d'ADN positif à la position 79 613 592 pb sur le chromosome 16.

8. Procédé selon l'une quelconque des revendications précédentes, dans lequel la génération de données génétiques comprend :
(a) le séquençage de l'ADN chromosomique, ou de copies amplifiées de celui-ci, le séquençage comprenant éventuellement un séquençage de nouvelle génération, et/ou
(b) une étape d'amplification, telle qu'une réaction en chaîne par polymérase.

9. Procédé comprenant : la détection d'un homozygote négatif pour un haplotype de DM dans un échantillon d'ADN génomique provenant d'un taureau, et l'identification du taureau comme étant apte à la reproduction sélective avec une femelle bovine, l'haplotype de DM comprenant :
(i) un ou plusieurs des allèles de la portion ombrée de la figure 5, la portion ombrée de la figure 5 correspondant aux positions 78 732 954 à 80 748 266 pb du chromosome 16 bovin, et/ou
(ii) un T sur le brin d'ADN positif à la position 79 613 592 pb sur le chromosome 16.

10. Procédé selon la revendication 9, dans lequel :
(a) la femelle est également homozygote négative pour l'haplotype de DM, ou
(b) le taureau est un taureau Holstein, un taureau croisé Holstein ou un taureau d'une autre race ayant des ancêtres Holstein, et la femelle bovine est une vache ou une génisse Holstein, une vache ou une génisse croisée Holstein, ou une vache ou une génisse d'une autre race ayant des ancêtres Holstein.

11. Procédé *in vitro*, le procédé comprenant : la détection d'un haplotype de DM homozygote négatif dans un échantillon comprenant un échantillon d'ADN génomique prélevé sur une femelle bovine ou un embryon issu de la femelle bovine, identifiant ainsi les ovocytes de la femelle comme étant adaptés à la production d'embryons *in vitro*, l'haplotype de DM comprenant :
(i) un ou plusieurs des allèles de la portion ombrée de la figure 5, la portion ombrée de la figure 5 correspondant aux positions 78 732 954 à 80 748 266 pb du chromosome 16 bovin, et/ou
(ii) un T sur le brin d'ADN positif à la position 79 613 592 pb sur le chromosome 16.

12. Procédé comprenant : la détermination de l'haplotype de DM sur un échantillon d'ADN génomique provenant d'un taureau et d'une vache, et l'identification d'une femelle homozygote négative pour l'haplotype de DM comme étant adaptée à l'accouplement avec un taureau positif pour l'haplotype de DM, l'haplotype de DM comprenant :
(i) un ou plusieurs des allèles de la portion ombrée de la figure 5, la portion ombrée de la figure 5 correspondant aux positions 78 732 954 à 80 748 266 pb du chromosome 16 bovin, et/ou
(ii) un T sur le brin d'ADN positif à la position 79 613 592 pb sur le chromosome 16.

13. Procédé comprenant : la détermination de l'haplotype de DM à partir d'un échantillon d'ADN génomique prélevé sur un taureau, et l'identification d'un taureau homozygote négatif pour l'haplotype de DM et d'une femelle positive pour l'haplotype de DM comme étant adaptés à la sélection corrective, l'haplotype de DM comprenant :
(i) un ou plusieurs des allèles de la portion ombrée de la figure 5, la portion ombrée de la figure 5 correspondant aux positions 78 732 954 à 80 748 266 pb du chromosome 16 bovin, et/ou
(ii) un T sur le brin d'ADN positif à la position 79613592 pb sur le chromosome 16.

14. Composition, kit ou système comprenant : au moins une molécule d'acide nucléique, la molécule d'acide nucléique comprenant la SEQ ID N° : 1, la SEQ ID N° : 2 ou un fragment de SEQ ID N° : 1 ou de SEQ ID N° : 2, le fragment comprenant le SNP de DM et au moins 10 nucléotides en 5' ou au moins 10 nucléotides en 3' de la position du SNP de DM.

15. Composition, kit ou système selon la revendication 14, dans lequel :
(a) la molécule d'acide nucléique est fixée à un vecteur solide,
(b) la composition, le kit ou le système comprend en outre une enzyme présentant une activité polymérase, l'enzyme comprenant éventuellement une Taq polymérase, ou
(c) la molécule d'acide nucléique comprend un marqueur détectable.
